# EUROPEAN PATENT APPLICATION

(11) **EP 4 044 311 A1**
(43) Date of publication of application: **17.08.2022**
(21) Application number: 20873410.3
(22) Date of filing: 30.09.2020
(51) Int. Cl.: H01M 10/0567, H01G 11/64, H01M 10/052, H01M 10/054, H01M 10/0568

(54) **NON-AQUEOUS ELECTROLYTE SOLUTION, AND NON-AQUEOUS ELECTROLYTE SECONDARY BATTERY**

(30) Priority: 07.10.2019 JP 2019184550; 07.10.2019 JP 2019184551
(71) Applicant: Mitsubishi Chemical Corporation, Chiyoda-ku Tokyo 100-8251 (JP); MU Ionic Solutions Corporation, Tokyo 100-8251 (JP)
(72) Inventor: WATANABE, Aiko, Tokyo 100-8251 (JP); NAKAZAWA, Eiji, Tokyo 100-8251 (JP)
(74) Representative: Vossius & Partner Patentanwälte Rechtsanwälte mbB
(86) International application number: PCT/JP2020/037323
(87) International publication number: WO 2021/070717

(57) **Abstract**

Provided are: a non-aqueous electrolyte solution with which a non-aqueous electrolyte secondary battery having both excellent durability and excellent charging characteristics can be provided; and a non-aqueous electrolyte secondary battery including the same. The non-aqueous electrolyte solution contains: a non-aqueous solvent: a compound represented by the following Formula (1); and at least one heteroatom-containing lithium salt selected from the group consisting of (A) a lithium salt having an F-S bond, (B) a lithium salt having an oxalic acid skeleton, and (C) a lithium salt having P=O and P-F bonds, and the content of the heteroatom-containing lithium salt is 0.001% by mass or more and 5% by mass or less: 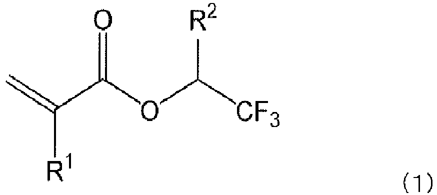 (wherein, R¹ represents a hydrogen atom or a methyl group, and R² represents a hydrogen atom, or a hydrocarbon group having 1 to 5 carbon atoms and optionally containing a halogen atom).

## Description

### TECHNICAL FIELD

The present invention relates to a non-aqueous electrolyte solution and a non-aqueous electrolyte secondary battery. More particularly, the present invention relates to: a non-aqueous electrolyte solution with which a non-aqueous electrolyte secondary battery having both excellent durability and excellent charging characteristics can be provided; and a non-aqueous electrolyte secondary battery.

### BACKGROUND ART

In recent years, the application and the usage of non-aqueous electrolyte secondary batteries such as lithium secondary batteries have been rapidly expanded. With regard to the application, non-aqueous electrolyte secondary batteries have been widely put into practical use ranging from power sources of mobile phones, laptop computers and the like to vehicle-mounted power sources for driving automobiles and the like. Under such circumstances, in recent years, there is an increasing demand in terms of the charging characteristics, such as a reduction in the charging time by rapid charging.

A variety of technologies have been proposed for improvement of the charging characteristics of non-aqueous electrolyte secondary batteries. For example, Patent Document 1 discloses a technology of improving the high-current characteristics by using lithium-titanium composite oxide particles having a specific average pore diameter as a negative electrode active material. Further, Patent Document 2 discloses a technology of improving the rapid charging characteristics by arranging solid particles between an active material layer and a separator. Moreover, Patent Document 3 discloses a technology that can improve the rapid charging characteristics by carrying out charging in a stepwise manner.

Meanwhile, durability typified by cycle characteristics and the like is one of the basic characteristics required for non-aqueous electrolyte secondary batteries. For improvement of the durability of a non-aqueous electrolyte secondary battery, it has been proposed to use an additive in a non-aqueous electrolyte solution. For example, Patent Document 4 discloses a technology that can improve the cycle characteristics by incorporating an unsaturated carboxylic acid ester into a non-aqueous electrolyte solution.

### RELATED ART DOCUMENTS

### PATENT DOCUMENTS

[Patent Document 1] Japanese Laid-open Patent Application (Kokai) No. 2007-18883
[Patent Document 2] Japanese Laid-open Patent Application (Kokai) No. 2015-138597
[Patent Document 3] Japanese Laid-open Patent Application (Kokai) No. H07-296853
[Patent Document 4] Japanese Laid-open Patent Application (Kokai) No. 2012-43632

### SUMMARY OF THE INVENTION

### PROBLEMS TO BE SOLVED BY THE INVENTION

Durability and charging characteristics are strongly demanded properties in non-aqueous electrolyte secondary batteries; however, according to the investigation by the present inventors, a problem was found in that an improvement in the durability and an improvement in the charging characteristics by the use of an additive in a non-aqueous electrolyte solution are in a conflicting relationship. Generally speaking, an improvement in the durability by an additive is brought about by inhibition of side reactions with an electrolyte solution through a specific action of the additive to an active material or formation of a surface coating film, and these actions on the surface cause an increase in the resistance at the electrode interface, resulting in deterioration of the charging characteristics. Nevertheless, in Patent Document 4, no specific evaluation or verification was made with regard to the charging characteristics, and the investigation by the present inventors revealed that the charging characteristics are yet to be satisfactory. The present invention was made in view of the above-described background, and an object of the present invention is to provide: a non-aqueous electrolyte solution with which a non-aqueous electrolyte secondary battery having both excellent durability and excellent charging characteristics, which are in a conflicting relationship, can be provided; and a non-aqueous electrolyte secondary battery including the non-aqueous electrolyte solution.

### MEANS FOR SOLVING THE PROBLEMS

The present inventors intensively studied to solve the above-described problems and consequently discovered that the problems can be solved by incorporating a fluorine-containing carboxylic acid ester compound having an acryloyl group or a methacryloyl group as a partial structure along with a specific heteroatom-containing lithium salt into a non-aqueous electrolyte solution used in a non-aqueous electrolyte secondary battery, thereby completing an invention A. That is, the gist of the present invention A is as follows.
[A1] A non-aqueous electrolyte solution, comprising:
   a non-aqueous solvent;
   a compound represented by the following Formula (1); and
   at least one heteroatom-containing lithium salt selected from the group consisting of (A) a lithium salt having an F-S bond, (B) a lithium salt having an oxalic acid skeleton, and (C) a lithium salt having P=O and P-F bonds,
   wherein the content of the heteroatom-containing lithium salt is 0.001% by mass or more and 5% by mass or less: 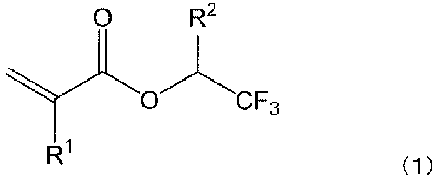
   (wherein, R¹ represents a hydrogen atom or a methyl group, and R² represents a hydrogen atom, a halogen atom, or a hydrocarbon group having 1 to 5 carbon atoms and optionally containing a halogen atom).
[A2] The non-aqueous electrolyte solution according to [A1], wherein the content of the compound represented by Formula (1) is 0.001% by mass or more and 20% by mass or less.
[A3] The non-aqueous electrolyte solution according to [A1] or [A2], wherein the content of the heteroatom-containing lithium salt is 0.001% by mass or more and 3% by mass or less.
[A4] The non-aqueous electrolyte solution according to any one of [A1] to [A3], further comprising at least one selected from the group consisting of LiPF₆, LiBF₄, LiClO₄, and Li(CF₃SO₂)₂N.
[A5] The non-aqueous electrolyte solution according to any one of [A1] to [A4], further comprising a cyclic carbonate compound having an unsaturated carbon-carbon bond, or a cyclic carbonate compound having a fluorine atom.
[A6] A non-aqueous electrolyte secondary battery, comprising:
   a negative electrode and a positive electrode that are capable of occluding and releasing metal ions; and
   a non-aqueous electrolyte solution,
   wherein the non-aqueous electrolyte solution is the non-aqueous electrolyte solution according to any one of [A1] to [A5] .

The present inventors intensively studied to solve the above-described problems and consequently discovered that the problems can be solved by incorporating a fluorine-containing carboxylic acid ester compound having an acryloyl group or a methacryloyl group as a partial structure along with a specific nitrogen atom-containing compound into a non-aqueous electrolyte solution used in a non-aqueous electrolyte secondary battery, thereby completing an invention B. That is, the gist of the present invention is as follows.
[B1] A non-aqueous electrolyte solution, comprising:
   a non-aqueous solvent;
   a lithium salt as an electrolyte; and
   a compound represented by Formula (2), and at least one of a compound represented by Formula (3) and an isocyanate compound: 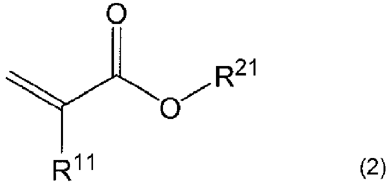
   (wherein, R¹¹ represents a hydrogen atom or a methyl group, and R²¹ represents a fluorine atom-containing hydrocarbon group having 1 to 10 carbon atoms) 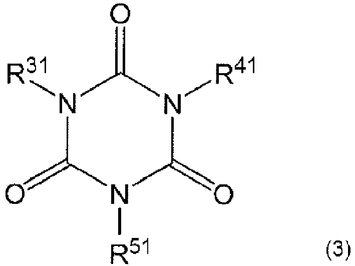
   (wherein, R³¹ to R⁵¹ may be the same or different from each other, and each represent an optionally substituted organic group having 1 to 20 carbon atoms).
[B2] The non-aqueous electrolyte solution according to [B1], wherein the content of the compound represented by Formula (2) is 0.001% by mass or more and 20% by mass or less.
[B3] The non-aqueous electrolyte solution according to [B1] or [B2], wherein the content of the compound represented by Formula (3) and/or the isocyanate compound is 0.001% by mass or more and 20% by mass or less.
[B4] A non-aqueous electrolyte secondary battery, comprising:
   a negative electrode and a positive electrode that are capable of occluding and releasing metal ions; and
   a non-aqeuous electrolyte solution,
   wherein the non-aqueous electrolyte solution is the non-aqueous electrolyte solution according to any one of [B1] to [B3].

### EFFECTS OF THE INVENTION

According to the present invention, the followings can be provided: a non-aqueous electrolyte solution with which a non-aqueous electrolyte secondary battery having both excellent durability and excellent charging characteristics can be provided; and a non-aqueous electrolyte secondary battery including the non-aqueous electrolyte solution.

### MODE FOR CARRYING OUT THE INVENTION

The present invention will now be described in detail. The following descriptions are merely examples (representative examples) of the present invention, and the present invention is not limited thereto. Further, the present invention can be carried out with any modification within the gist of the present invention.

### [Non-aqueous Electrolyte Solution A]

The non-aqueous electrolyte solution according to one embodiment of the present invention A contains: a non-aqueous solvent; a compound represented by the following Formula (1) (hereinafter, may be referred to as "compound (1)"); and at least one heteroatom-containing lithium salt selected from the group consisting of (A) a lithium salt having an F-S bond, (B) a lithium salt having an oxalic acid skeleton, and (C) a lithium salt having P=O and P-F bonds: 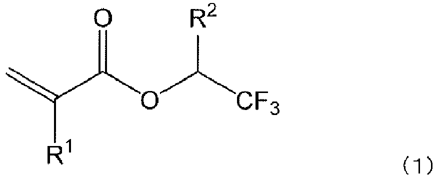 (wherein, R¹ represents a hydrogen atom or a methyl group, and R² represents a hydrogen atom, a halogen atom, or a hydrocarbon group having 1 to 5 carbon atoms and optionally containing a halogen atom).

The non-aqueous electrolyte solution of the present invention A has an effect of exerting both excellent durability and excellent charging characteristics. The reason why the present invention A has this effect is not clear; however, it is presumed that the effect is attributed to the following mechanism. The acryloyl group or the methacryloyl group contained in the compound (1) as a partial structure undergoes a polymerization reaction with an anion species generated in the vicinity of a negative electrode, and an underlayer to which fluorine atom-containing carboxylic acid ester groups are bound is thereby formed on the negative electrode. After the formation of this underlayer, the lithium salt having a heteroatom-containing specific skeleton ((A) lithium salt having an F-S bond, (B) lithium salt having an oxalic acid skeleton, or (C) lithium salt having P=O and P-F bonds) undergoes a reductive decomposition reaction on the negative electrode to form a coating film containing lithium atoms and heteroatoms on the underlayer. In this process, since the coating film containing lithium is formed on the underlayer containing a fluorine atom-containing carboxylic acid ester, fluorine of the underlayer and lithium of the coating film react with each other to form a coating film containing a large amount of lithium fluoride. This coating film containing a large amount of lithium fluoride is known to have a high durability. In the present invention A, it is believed that excellent durability is obtained because of the formation of the coating film containing a large amount of lithium fluoride. In addition, it is believed that, since the coating film contains heteroatoms originated from the lithium salt, the mobility of lithium ions inside the coating film is enhanced, so that the charging characteristics are improved. In other words, it is presumed that a compound having a fluorine atom and a polymerizable group forms an underlayer, and a heteroatom-containing specific lithium salt forms a coating film on the underlayer, as a result of which the underlayer and the coating film react with each other to synergistically form a favorable coating film that has both satisfactory durability and satisfactory charging characteristics.

### <A1. Compound (1) Represented by Formula (1)>

The non-aqueous electrolyte solution of the present invention A contains a compound (1) represented by Formula (1). In Formula (1), R¹ represents a hydrogen atom or a methyl group, and R² represents a hydrogen atom, a halogen atom, or a hydrocarbon group having 1 to 5 carbon atoms and optionally containing a halogen atom. Examples of the halogen atom include a fluorine atom, a chlorine atom, and a bromine atom.

R² is preferably a hydrogen atom; a fluorine atom; a hydrocarbon group, such as a methyl group, an ethyl group, or a propyl group; or a fluorine atom-containing hydrocarbon group, such as a trifluoromethyl group or a trifluoroethyl group, more preferably a hydrogen atom or a perfluoroalkyl group, particularly preferably a hydrogen atom or a trifluoromethyl group.

Specific examples of the compound (1) include 2,2,2-trifluoroethyl acrylate, 2,2,2-trifluoroethyl methacrylate, 1,1,1,3,3,3-hexafluoroisopropyl acrylate, and 1,1,1,3,3,3-hexafluoroisopropyl methacrylate. Thereamong, 2,2,2-trifluoroethyl acrylate is preferred since it has an optimum reaction potential.

The compound (1) is characterized by being a fluorine-containing carboxylic acid ester compound having an acryloyl group or a methacryloyl group as a partial structure. It is believed that the acryloyl group or the methacryloyl group is a partial structure required for the formation of an underlayer on a negative electrode by polymerization reaction, and that fluorine atoms are required for introducing a large amount of lithium fluoride to a coating film. In addition, it is believed that the compound (1) is required to have a preferred reaction potential for a specific heteroatom-containing lithium salt, and that an excessively high reaction potential causes decomposition of the compound (1) itself while an excessively low reaction potential cannot generate a synergistic reaction with a specific heteroatom-containing lithium salt. Among fluorine-containing carboxylic acid ester compounds having an acryloyl group or a methacryloyl group as a partial structure, one having a structure corresponding to the compound (1) is believed to have a preferred reaction potential for a specific heteroatom-containing lithium salt.

The content of the compound (1) in the non-aqueous electrolyte solution is not particularly restricted as long as the effects of the present invention A are not markedly impaired. Specifically, a lower limit value of the content of the compound (1) in the non-aqueous electrolyte solution is preferably not less than 0.001% by mass, more preferably not less than 0.05% by mass, still more preferably not less than 0.1% by mass, with respect to a total amount of the non-aqueous electrolyte solution. Meanwhile, an upper limit value is preferably 20% by mass or less, more preferably 10% by mass or less, still more preferably 5% by mass or less, with respect to a total amount of the non-aqueous electrolyte solution. When the concentration of the compound (1) is in the above-described preferred range, an effect of improving the durability and the charging characteristics is more likely to be exerted without deterioration of other battery performance. A method for identifying the compound (1) and measuring the content thereof is not particularly restricted, and any known method may be selected as appropriate. Examples thereof include gas chromatography and nuclear magnetic resonance (NMR) spectroscopy. As long as the compound (1) is contained in the non-aqeuous electrolyte solution, the compound (1) encompasses a mode of being added to the non-aqueous electrolyte solution and a mode of being generated in the non-aqueous electrolyte solution or in a non-aqueous electrolyte battery during its operation.

### <A2. Specific Heteroatom-Containing Lithium Salt>

The non-aqueous electrolyte solution of the present invention A contains at least one heteroatom-containing lithium salt selected from the group consisting of (A) a lithium salt having an F-S bond, (B) a lithium salt having an oxalic acid skeleton, and (C) a lithium salt having P=O and P-F bonds. Among these lithium salts, (A) a lithium salt having an F-S bond or (B) a lithium salt having an oxalic acid skeleton is preferred, and (A) a lithium salt having an F-S bond is more preferred.

The content of the heteroatom-containing lithium salt selected from the group consisting of (A) a lithium salt having an F-S bond, (B) a lithium salt having an oxalic acid skeleton, and (C) a lithium salt having P=O and P-F bonds in the non-aqueous electrolyte solution is not particularly restricted as long as the effects of the present invention A are not markedly impaired. Specifically, a lower limit value of the content of the heteroatom-containing lithium salt in the non-aqueous electrolyte solution is preferably not less than 0.001% by mass, more preferably not less than 0.05% by mass, still more preferably not less than 0.1% by mass, with respect to a total amount of the non-aqueous electrolyte solution. Meanwhile, an upper limit value is preferably 20% by mass or less, more preferably 10% by mass or less, still more preferably 5% by mass or less, especially preferably 3% by mass or less, particularly preferably 2% by mass or less, with respect to a total amount of the non-aqueous electrolyte solution. When the concentration of the heteroatom-containing lithium salt is in the above-described preferred range, an effect of improving the durability and the charging characteristics is more likely to be exerted without deterioration of other battery performance.

Further, two or more of (A) a lithium salt having an F-S bond, (B) a lithium salt having an oxalic acid skeleton, and (C) a lithium salt having P=O and P-F bonds may be used in combination, and it is particularly preferred to use a combination of (A) a lithium salt having an F-S bond and (C) a lithium salt having P=O and P-F bonds. When the non-aqueous electrolyte solution contains two or more heteroatom-containing lithium salts, a total amount thereof preferably satisfies the above-described range.

### <A2-1. (A) Lithium Salt Having F-S Bond>

The (A) lithium salt having an F-S bond (hereinafter, may be referred to as "heteroatom-containing lithium salt (A)") that is used in the present invention A is not particularly restricted as long as it is a lithium salt that has an F-S bond in its molecule, and any such lithium salt can be used as long as the effects of the present invention A are not markedly impaired.

Examples of the heteroatom-containing lithium salt (A) include, but not particularly limited to:
lithium fluorosulfonate (LiFSO₃);
fluorosulfonylimide lithium salts, such as lithium bis (fluorosulfonyl) imide (LiN(FSO₂)₂) and LiN(F_{S}O₂)(CF₃SO₂);
fluorosulfonylmethide lithium salts, such as LiC(FSO₂)₃; and
lithium fluorosulfonyl borates, such as LiBF₃(FSO₃) and LiB(FSO₂)₄.

The heteroatom-containing lithium salt (A) may be used singly, or in combination of two or more thereof.

Thereamong, LiFSO₃ or LiN(FSO₂)₂ is preferred, and LiFSO₃ is particularly preferred.

In the non-aqueous electrolyte solution of the present invention A, the heteroatom-containing lithium salt (A) is preferably used as an auxiliary electrolyte. That is, a lower limit value of the content of the heteroatom-containing lithium salt (A) is preferably not less than 0.01% by mass, more preferably not less than 0.05% by mass, still more preferably not less than 0.1% by mass, based on a total amount of the non-aqueous electrolyte solution. Meanwhile, an upper limit value is preferably 20% by mass or less, more preferably 10% by mass or less, still more preferably 5% by mass or less, especially preferably 3% by mass or less, particularly preferably 2% by mass or less, based on a total amount of the non-aqueous electrolyte solution. A method for identifying the heteroatom-containing lithium salt (A) and measuring the content thereof is not particularly restricted, and any known method may be selected as appropriate. Examples thereof include ion chromatography and nuclear magnetic resonance (NMR) spectroscopy.

When the concentration of the heteroatom-containing lithium salt (A) is in the above-described preferred range, an effect of improving the durability and the charging characteristics is more likely to be exerted without deterioration of other battery performance.

### <A2-2. (B) Salt Having Oxalic Acid Skeleton>

The (B) salt having an oxalic acid skeleton (hereinafter, may be referred to as "heteroatom-containing lithium salt (B)") that is used in the present invention A is not particularly restricted as long as it is a salt that has an oxalic acid skeleton in its molecule, and any such salt can be used as long as the effects of the present invention are not markedly impaired.

Examples of the heteroatom-containing lithium salt (B) include, but not particularly limited to: lithium bis(oxalato)borate (LiBOB), lithium difluoro(oxalato)borate (LiDFOB), lithium tetrafluoro(oxalato)phosphate, and lithium difluoro-bis(oxalato)phosphate (LiDFOP). The heteroatom-containing lithium salt (B) may be used singly, or in combination of two or more thereof.

Thereamong, LiBOB, LiDFOB, and LiDFOP are more preferred, and LiBOB is particularly preferred.

In the non-aqueous electrolyte solution of the present invention A, the content of the heteroatom-containing lithium salt (B) is not particularly restricted as long as the effects of the present invention A are not markedly impaired, and the heteroatom-containing lithium salt (B) is preferably used as an auxiliary electrolyte. That is, a lower limit value of the content of the heteroatom-containing lithium salt (B) is preferably not less than 0.01% by mass, more preferably not less than 0.05% by mass, still more preferably not less than 0.1% by mass, based on a total amount of the non-aqueous electrolyte solution. Meanwhile, an upper limit value is preferably 20% by mass or less, more preferably 10% by mass or less, still more preferably 5% by mass or less, especially preferably 3% by mass or less, particularly preferably 2% by mass or less, based on a total amount of the non-aqueous electrolyte solution. A method for identifying the heteroatom-containing lithium salt (B) and measuring the content thereof is not particularly restricted, and any known method may be selected as appropriate. Examples thereof include ion chromatography and nuclear magnetic resonance (NMR) spectroscopy.

When the concentration of the heteroatom-containing lithium salt (B) is in the above-described preferred range, an effect of improving the durability and the charging characteristics is more likely to be exerted without deterioration of other battery performance.

### <A2-3. (C) Lithium Salt Having P=O and P-F Bonds>

The (C) lithium salt having P=O and P-F bonds (hereinafter, may be referred to as "heteroatom-containing lithium salt (C)") that is used in the present invention A is not particularly restricted as long as it is a lithium salt that has P=O and P-F bonds in its molecule, and any such lithium salt can be used as long as the effects of the present invention A are not markedly impaired.

Examples of the heteroatom-containing lithium salt (C) include, but not particularly limited to: lithium difluorophosphate (LiPO₂F₂) and lithium fluorophosphate (Li₂PO₃F). The heteroatom-containing lithium salt (C) may be used singly, or in combination of two or more thereof.

Thereamong, LiPO₂F₂ is particularly preferred.

In the non-aqueous electrolyte solution of the present invention A, the content of the heteroatom-containing lithium salt (C) is not particularly restricted as long as the effects of the present invention are not markedly impaired, and the heteroatom-containing lithium salt (C) is preferably used as an auxiliary electrolyte. That is, a lower limit value of the content of the heteroatom-containing lithium salt (C) is preferably not less than 0.01% by mass, more preferably not less than 0.05% by mass, still more preferably not less than 0.1% by mass, based on a total amount of the non-aqueous electrolyte solution. Meanwhile, an upper limit value is preferably 20% by mass or less, more preferably 10% by mass or less, still more preferably 5% by mass or less, especially preferably 3% by mass or less, particularly preferably 2% by mass or less, based on a total amount of the non-aqueous electrolyte solution. A method for identifying the heteroatom-containing lithium salt (C) and measuring the content thereof is not particularly restricted, and any known method may be selected as appropriate. Examples thereof include ion chromatography and nuclear magnetic resonance (NMR) spectroscopy.

When the concentration of the heteroatom-containing lithium salt (C) is in the above-described preferred range, an effect of improving the durability and the charging characteristics is more likely to be exerted without deterioration of other battery performance.

### <A3. Non-aqueous Solvent>

Similarly to a general non-aqueous electrolyte solution, the non-aqueous electrolyte solution of the present invention A usually contains, as its main component, a non-aqueous solvent that dissolves the above-described electrolytes. The non-aqueous solvent is not particularly restricted, and any known organic solvent can be used. The organic solvent is not particularly restricted; however, it is preferably, for example, at least one selected from a saturated cyclic carbonate, a linear carbonate, a linear carboxylic acid ester, a cyclic carboxylic acid ester, an ether-based compound, and a sulfone-based compound. These non-aqueous solvents may be used singly, or in combination of two or more thereof.

<A3-1. Saturated Cyclic Carbonate>

The saturated cyclic carbonate is usually, for example, one having an alkylene group having 2 to 4 carbon atoms. Examples thereof include ethylene carbonate, propylene carbonate, and butylene carbonate. Thereamong, ethylene carbonate or propylene carbonate is preferred from the standpoint of attaining an improvement in the battery characteristics that is attributed to an increase in the degree of lithium ion dissociation. Any of these saturated cyclic carbonates may be used singly, or two or more thereof may be used in any combination at any ratio.

The content of the saturated cyclic carbonate is not particularly restricted and may be set arbitrarily as long as the effects of the present invention A are not markedly impaired; however, it is usually not less than 3% by volume, preferably not less than 5% by volume, in 100% by volume of the non-aqueous solvent. By controlling the content of the saturated cyclic carbonate to be in this range, a decrease in the electrical conductivity of the non-aqueous electrolyte solution caused by a reduction in the dielectric constant is avoided, so that the high-current discharge characteristics of a non-aqueous electrolyte secondary battery, the stability to a negative electrode, and the cycle characteristics are all likely to be obtained in favorable ranges. Meanwhile, an upper limit of the content of the saturated cyclic carbonate is usually 90% by volume or less, preferably 85% by volume or less, more preferably 80% by volume or less, in 100% by volume of the non-aqueous solvent. By controlling the content of the saturated cyclic carbonate to be in this range, the viscosity of the non-aqueous electrolyte solution is kept in an appropriate range and a reduction in the ionic conductivity is inhibited, as a result of which not only the input-output characteristics of a non-aqueous electrolyte secondary battery can be further improved but also the durability, such as cycle characteristics and storage characteristics, can be further enhanced, which is preferred.

### <A3-2. Linear Carbonate>

As the linear carbonate, one having 3 to 7 carbon atoms is preferred. Specific examples of the linear carbonate having 3 to 7 carbon atoms include dimethyl carbonate, diethyl carbonate, di-*n*-propyl carbonate, diisopropyl carbonate, *n-*propyl isopropyl carbonate, ethyl methyl carbonate, methyl-*n-*propyl carbonate, *n*-butyl methyl carbonate, isobutyl methyl carbonate, *t*-butyl methyl carbonate, ethyl-*n*-propyl carbonate, *n*-butyl ethyl carbonate, isobutyl ethyl carbonate, and *t*-butyl ethyl carbonate. Thereamong, dimethyl carbonate, diethyl carbonate, di-*n*-propyl carbonate, diisopropyl carbonate, *n-*propyl isopropyl carbonate, ethyl methyl carbonate and methyl-*n*-propyl carbonate are preferred, and dimethyl carbonate, diethyl carbonate and ethyl methyl carbonate are particularly preferred.

Further, a fluorine atom-containing linear carbonate (hereinafter, may be simply referred to as "fluorinated linear carbonate") can be preferably used as well. The number of fluorine atoms in the fluorinated linear carbonate is not particularly restricted; however, it is usually 6 or less, preferably 4 or less. When the fluorinated linear carbonate has plural fluorine atoms, the fluorine atoms may be bound to the same carbon, or may be bound to different carbons. Examples of the fluorinated linear carbonate include fluorinated dimethyl carbonate derivatives, fluorinated ethyl methyl carbonate derivatives, and fluorinated diethyl carbonate derivatives.

Any of the above-described linear carbonates may be used singly, or two or more thereof may be used in any combination at any ratio.

The content of the linear carbonate is not particularly restricted; however, it is usually not less than 15% by volume, preferably not less than 20% by volume, more preferably not less than 25% by volume, but usually 90% by volume or less, preferably 85% by volume or less, more preferably 80% by volume or less, in 100% by volume of the non-aqueous solvent. By controlling the content of the linear carbonate to be in this range, not only the viscosity of the non-aqueous electrolyte solution is kept in an appropriate range and a reduction in the ionic conductivity is inhibited, but also a decrease in the electrical conductivity caused by a reduction in the dielectric constant of the non-aqueous electrolyte solution can be avoided. As a result, the input-output characteristics and the charge-discharge rate characteristics of a non-aqueous electrolyte secondary battery are likely to be attained in favorable ranges.

### <A3-3. Linear Carboxylic Acid Ester>

Examples of the linear carboxylic acid ester include those having a total of 3 to 7 carbon atoms in their respective structures. Specific examples of such linear carboxylic acid esters include methyl acetate, ethyl acetate, *n*-propyl acetate, isopropyl acetate, *n*-butyl acetate, isobutyl acetate, *t*-butyl acetate, methyl propionate, ethyl propionate, *n*-propyl propionate, isopropyl propionate, *n*-butyl propionate, isobutyl propionate, *t*-butyl propionate, methyl butyrate, ethyl butyrate, *n*-propyl butyrate, isopropyl butyrate, methyl isobutyrate, ethyl isobutyrate, *n*-propyl isobutyrate, and isopropyl isobutyrate. Thereamong, for example, methyl acetate, ethyl acetate, *n*-propyl acetate, *n*-butyl acetate, methyl propionate, ethyl propionate, n-propyl propionate, isopropyl propionate, methyl butyrate, and ethyl butyrate are preferred from the standpoint of improving the ionic conductivity through a reduction in the viscosity and inhibiting battery swelling during durability tests for cycle operation and storage.

The content of the linear carboxylic acid ester is not particularly restricted; however, it is usually not less than 3% by volume, preferably not less than 5% by volume, more preferably not less than 10% by volume, but usually 30% by volume or less, preferably 20% by volume or less, more preferably 15% by volume or less, in 100% by volume of the non-aqueous solvent. By controlling the content of the linear carboxylic acid ester to be in this range, the viscosity of the non-aqueous electrolyte solution is kept in an appropriate range and a reduction in the ionic conductivity is inhibited, as a result of which the output characteristics of a non-aqueous electrolyte secondary battery are likely to be attained in favorable ranges.

### <A3-4. Cyclic Carboxylic Acid Ester>

Examples of the cyclic carboxylic acid ester include those having a total of 3 to 12 carbon atoms in their respective structures. Specific examples of such cyclic carboxylic acid esters include *γ*-butyrolactone, *γ-*valerolactone, *γ*-caprolactone, and *ε*-caprolactone. Thereamong, *γ*-butyrolactone is particularly preferred from the standpoint of attaining an improvement in the battery characteristics that is attributed to an increase in the degree of lithium ion dissociation.

The content of the cyclic carboxylic acid ester is not particularly restricted; however, it is usually not less than 3% by volume, preferably not less than 5% by volume, more preferably not less than 10% by volume, but usually 30% by volume or less, preferably 20% by volume or less, more preferably 15% by volume or less, in 100% by volume of the non-aqueous solvent. By controlling the content of the cyclic carboxylic acid ester to be in this range, the viscosity of the non-aqueous electrolyte solution is kept in an appropriate range and a reduction in the ionic conductivity is inhibited, as a result of which the output characteristics of a non-aqueous electrolyte secondary battery are likely to be attained in favorable ranges.

### <A3-5. Ether-Based Compound>

The ether-based compound is preferably a linear ether having 3 to 10 carbon atoms, or a cyclic ether having 3 to 6 carbon atoms.

Examples of the linear ether having 3 to 10 carbon atoms include diethyl ether, di(2-fluoroethyl)ether, di(2,2-difluoroethyl)ether, di(2,2,2-trifluoroethyl)ether, ethyl(2-fluoroethyl)ether, ethyl(2,2,2-trifluoroethyl)ether, ethyl(1,1,2,2-tetrafluoroethyl)ether, (2-fluoroethyl)(2,2,2-trifluoroethyl)ether, (2-fluoroethyl)(1,1,2,2-tetrafluoroethyl)ether, (2,2,2-trifluoroethyl)(1,1,2,2-tetrafluoroethyl)ether, ethyl-*n*-propyl ether, ethyl(3-fluoro-*n*-propyl)ether, ethyl(3,3,3-trifluoro-*n*-propyl)ether, ethyl(2,2,3,3-tetrafluoro-*n*-propyl)ether, ethyl(2,2,3,3,3-pentafluoro-*n*-propyl)ether, 2-fluoroethyl-*n*-propyl ether, (2-fluoroethyl)(3-fluoro-*n*-propyl)ether, (2-fluoroethyl)(3,3,3-trifluoro-*n*-propyl)ether, (2-fluoroethyl)(2,2,3,3-tetrafluoro-*n*-propyl)ether, (2-fluoroethyl)(2,2,3,3,3-pentafluoro-*n-*propyl)ether, 2,2,2-trifluoroethyl-*n*-propyl ether, (2,2,2-trifluoroethyl)(3-fluoro-*n*-propyl)ether, (2,2,2-trifluoroethyl)(3,3,3-trifluoro-*n*-propyl)ether, (2,2,2-trifluoroethyl)(2,2,3,3-tetrafluoro-*n*-propyl)ether, (2,2,2-trifluoroethyl)(2,2,3,3,3-pentafluoro-*n*-propyl)ether, 1,1,2,2-tetrafluoroethyl-*n*-propyl ether, (1,1,2,2-tetrafluoroethyl)(3-fluoro-*n*-propyl)ether, (1,1,2,2-tetrafluoroethyl)(3,3,3-trifluoro-*n*-propyl)ether, (1,1,2,2-tetrafluoroethyl)(2,2,3,3-tetrafluoro-*n*-propyl)ether, (1,1,2,2-tetrafluoroethyl)(2,2,3,3,3-pentafluoro-*n*-propyl)ether, di-*n-*propyl ether, (*n*-propyl)(3-fluoro-n-propyl)ether, (*n-*propyl)(3,3,3-trifluoro-*n*-propyl)ether, (*n*-propyl)(2,2,3,3-tetrafluoro-*n*-propyl)ether, (*n*-propyl)(2,2,3,3,3-pentafluoro-*n*-propyl)ether, di(3-fluoro-*n*-propyl)ether, (3-fluoro-*n-*propyl)(3,3,3-trifluoro-*n*-propyl)ether, (3-fluoro-*n-*propyl)(2,2,3,3-tetrafluoro-*n*-propyl)ether, (3-fluoro-*n-*propyl)(2,2,3,3,3-pentafluoro-*n*-propyl)ether, di(3,3,3-trifluoro-*n*-propyl)ether, (3,3,3-trifluoro-*n*-propyl)(2,2,3,3-tetrafluoro-*n*-propyl)ether, (3,3,3-trifluoro-*n-*propyl)(2,2,3,3,3-pentafluoro-*n*-propyl)ether, di(2,2,3,3-tetrafluoro-*n*-propyl)ether, (2,2,3,3-tetrafluoro-*n-*propyl)(2,2,3,3,3-pentafluoro-*n*-propyl)ether, di(2,2,3,3,3-pentafluoro-*n*-propyl)ether, di-*n*-butyl ether, dimethoxymethane, methoxyethoxymethane, methoxy(2-fluoroethoxy)methane, methoxy(2,2,2-trifluoroethoxy)methane, methoxy(1,1,2,2-tetrafluoroethoxy)methane, diethoxymethane, ethoxy(2-fluoroethoxy)methane, ethoxy(2,2,2-trifluoroethoxy)methane, ethoxy(1,1,2,2-tetrafluoroethoxy)methane, di(2-fluoroethoxy)methane, (2-fluoroethoxy)(2,2,2-trifluoroethoxy)methane, (2-fluoroethoxy)(1,1,2,2-tetrafluoroethoxy)methane, di(2,2,2-trifluoroethoxy)methane, (2,2,2-trifluoroethoxy)(1,1,2,2-tetrafluoroethoxy)methane, di(1,1,2,2-tetrafluoroethoxy)methane, dimethoxyethane, methoxyethoxyethane, methoxy(2-fluoroethoxy)ethane, methoxy(2,2,2-trifluoroethoxy)ethane, methoxy(1,1,2,2-tetrafluoroethoxy)ethane, diethoxyethane, ethoxy(2-fluoroethoxy)ethane, ethoxy(2,2,2-trifluoroethoxy)ethane, ethoxy(1,1,2,2-tetrafluoroethoxy)ethane, di(2-fluoroethoxy)ethane, (2-fluoroethoxy) (2,2,2-trifluoroethoxy)ethane, (2-fluoroethoxy)(1,1,2,2-tetrafluoroethoxy)ethane, di(2,2,2-trifluoroethoxy)ethane, (2,2,2-trifluoroethoxy) (1,1,2,2-tetrafluoroethoxy)ethane, di(1,1,2,2-tetrafluoroethoxy)ethane, ethylene glycol di-*n-*propyl ether, ethylene glycol di-*n*-butyl ether, and diethylene glycol dimethyl ether.

Examples of the cyclic ether include tetrahydrofuran, 2-methyltetrahydrofuran, 3-methyltetrahydrofuran, 1,3-dioxane, 2-methyl-1,3-dioxane, 4-methyl-1,3-dioxane, 1,4-dioxane, and fluorinated compounds thereof.

Among the above-described ether-based compounds, dimethoxymethane, diethoxymethane, ethoxymethoxymethane, ethylene glycol di-*n-*propyl ether, ethylene glycol di-*n*-butyl ether, and diethylene glycol dimethyl ether are preferred since they have a high solvating capacity with lithium ions and thus improve the lithium ion dissociation. Particularly preferred are dimethoxymethane, diethoxymethane, and ethoxymethoxymethane since they have a low viscosity and provide a high ionic conductivity.

The content of the ether-based compound is not particularly restricted; however, it is usually not less than 1% by volume, preferably not less than 2% by volume, more preferably not less than 3% by volume, but usually 30% by volume or less, preferably 25% by volume or less, more preferably 20% by volume or less, in 100% by volume of the non-aqueous solvent. When the content of the ether-based compound is in this preferred range, an ionic conductivity-improving effect of ether, which is attributed to an increase in the degree of lithium ion dissociation and a reduction in the viscosity, is likely to be ensured. In addition, when a carbonaceous material is used as a negative electrode active material, the phenomenon of co-intercalation of a linear ether thereto along with lithium ions can be inhibited; therefore, the input-output characteristics and the charge-discharge rate characteristics can be attained in appropriate ranges.

### <A3-6. Sulfone-Based Compound>

The sulfone-based compound is not particularly restricted and may be a cyclic sulfone or a linear sulfone; however, it is preferably a cyclic sulfone having 3 to 6 carbon atoms, or a linear sulfone having 2 to 6 carbon atoms. The number of sulfonyl groups in one molecule of the sulfone-based compound is preferably 1 or 2.

Examples of the cyclic sulfone include: monosulfone compounds, such as trimethylene sulfones, tetramethylene sulfones, and hexamethylene sulfones; and disulfone compounds, such as trimethylene disulfones, tetramethylene disulfones, and hexamethylene disulfones. Thereamong, from the standpoints of the dielectric constant and the viscosity, tetramethylene sulfones, tetramethylene disulfones, hexamethylene sulfones and hexamethylene disulfones are more preferred, and tetramethylene sulfones (sulfolanes) are particularly preferred.

As the sulfolanes, sulfolane and sulfolane derivatives are preferred. As the sulfolane derivatives, those in which one or more of the hydrogen atoms bound to carbon atoms constituting a sulfolane ring are each substituted with a fluorine atom, an alkyl group, or a fluorine-substituted alkyl group are preferred.

Thereamong, for example, 2-methyl sulfolane, 3-methyl sulfolane, 2-fluorosulfolane, 3-fluorosulfolane, 2,2-difluorosulfolane, 2,3-difluorosulfolane, 2,4-difluorosulfolane, 2,5-difluorosulfolane, 3,4-difluorosulfolane, 2-fluoro-3-methyl sulfolane, 2-fluoro-2-methyl sulfolane, 3-fluoro-3-methyl sulfolane, 3-fluoro-2-methyl sulfolane, 4-fluoro-3-methyl sulfolane, 4-fluoro-2-methyl sulfolane, 5-fluoro-3-methyl sulfolane, 5-fluoro-2-methyl sulfolane, 2-fluoromethyl sulfolane, 3-fluoromethyl sulfolane, 2-difluoromethyl sulfolane, 3-difluoromethyl sulfolane, 2-trifluoromethyl sulfolane, 3-trifluoromethyl sulfolane, 2-fluoro-3-(trifluoromethyl)sulfolane, 3-fluoro-3-(trifluoromethyl)sulfolane, 4-fluoro-3-(trifluoromethyl)sulfolane, and 5-fluoro-3-(trifluoromethyl)sulfolane are preferred from the standpoint of attaining a high ionic conductivity and a high input/output.

Further, examples of the linear sulfone include dimethyl sulfone, ethyl methyl sulfone, diethyl sulfone, *n*-propyl methyl sulfone, *n*-propyl ethyl sulfone, di-*n*-propyl sulfone, isopropyl methyl sulfone, isopropyl ethyl sulfone, diisopropyl sulfone, *n*-butyl methyl sulfone, *n*-butyl ethyl sulfone, *t-*butyl methyl sulfone, *t*-butyl ethyl sulfone, monofluoromethyl methyl sulfone, difluoromethyl methyl sulfone, trifluoromethyl methyl sulfone, monofluoroethyl methyl sulfone, difluoroethyl methyl sulfone, trifluoroethyl methyl sulfone, pentafluoroethyl methyl sulfone, ethyl monofluoromethyl sulfone, ethyl difluoromethyl sulfone, ethyl trifluoromethyl sulfone, perfluoroethyl methyl sulfone, ethyl trifluoroethyl sulfone, ethyl pentafluoroethyl sulfone, di(trifluoroethyl) sulfone, perfluorodiethyl sulfone, fluoromethyl-n-propyl sulfone, difluoromethyl-*n*-propyl sulfone, trifluoromethyl-*n-*propyl sulfone, fluoromethyl isopropyl sulfone, difluoromethyl isopropyl sulfone, trifluoromethyl isopropyl sulfone, trifluoroethyl-*n*-propyl sulfone, trifluoroethyl isopropyl sulfone, pentafluoroethyl-*n*-propyl sulfone, pentafluoroethyl isopropyl sulfone, trifluoroethyl-*n*-butyl sulfone, trifluoroethyl-*t*-butyl sulfone, pentafluoroethyl-*n*-butyl sulfone, and pentafluoroethyl-*t*-butyl sulfone.

Thereamong, for example, dimethyl sulfone, ethyl methyl sulfone, diethyl sulfone, *n*-propyl methyl sulfone, isopropyl methyl sulfone, *n*-butyl methyl sulfone, *t*-butyl methyl sulfone, monofluoromethyl methyl sulfone, difluoromethyl methyl sulfone, trifluoromethyl methyl sulfone, monofluoroethyl methyl sulfone, difluoroethyl methyl sulfone, trifluoroethyl methyl sulfone, pentafluoroethyl methyl sulfone, ethyl monofluoromethyl sulfone, ethyl difluoromethyl sulfone, ethyl trifluoromethyl sulfone, ethyl trifluoroethyl sulfone, ethyl pentafluoroethyl sulfone, trifluoromethyl-*n-*propyl sulfone, trifluoromethyl isopropyl sulfone, trifluoroethyl-*n*-butyl sulfone, trifluoroethyl-*t*-butyl sulfone, trifluoromethyl-*n*-butyl sulfone, and trifluoromethyl-*t*-butyl sulfone are preferred from the standpoint of attaining a high ionic conductivity and a high input/output.

The content of the sulfone-based compound is not particularly restricted; however, it is usually not less than 0.3% by volume, preferably not less than 0.5% by volume, more preferably not less than 1% by volume, but usually 40% by volume or less, preferably 35% by volume or less, more preferably 30% by volume or less, in 100% by volume of the non-aqueous solvent. When the content of the sulfone-based compound is in this range, an electrolyte solution having excellent high-temperature storage stability tends to be obtained.

### <A4. Electrolytes>

### <A4-1. Lithium Salt Other Than Specific Heteroatom-Containing Lithium Salt>

The non-aqueous electrolyte solution of the present invention A may contain, as an electrolyte, at least one lithium salt other than the above-described specific heteroatom-containing lithium salt (hereinafter, also referred to as "other lithium salt"). The other lithium salt is not particularly restricted as long as it is one that is usually used in this type of application. The other lithium salt can be used as a main electrolyte or an auxiliary electrolyte; however, it is preferably used as a main electrolyte. Specific examples of the other lithium salt include the below-described lithium salts. The other lithium salt may be used singly, or in combination of two or more thereof.

Examples of the other lithium salt that can be used in the non-aqueous electrolyte solution of the present invention A include LiClO₄, LiBF₄, LiPF₆, LiAsF₆, LiTaF₆, LiCF₃SO₃, LiC₄F₉SO₃, Li(CF₃SO₂)₂N, Li(C₂F₅SO₂)₂N, Li(CF₃SO₂)₃C, LiBF₃(C₂F₅), LiB(C₆F₅)₄, and LiPF₃(C₂F₅)₃. Thereamong, the other lithium salt is preferably at least one selected from LiPF₆, LiBF₄, LiClO₄, and Li(CF₃SO₂)₂N, more preferably at least one selected from LiPF₆, LiBF₄, and Li(CF₃SO₂)₂N, particularly preferably LiPF₆. The above-exemplified lithium salts may be used singly, or in combination of two or more thereof.

In cases where the other lithium salt is used as a main salt, the concentration (content) thereof may be set arbitrarily as long as the effects of the present invention A are not markedly impaired; however, the concentration of the other lithium salt in the non-aqueous electrolyte solution is preferably not lower than 0.5 mol/L, more preferably not lower than 0.6 mol/L, still more preferably not lower than 0.7 mol/L, but preferably 3 mol/L or lower, more preferably 2 mol/L or lower, still more preferably 1.8 mol/L or lower. The content (% by mass) of the other lithium salt is preferably not less than 6% by mass, more preferably not less than 7% by mass, still more preferably not less than 8% by mass, but preferably 30% by mass or less, more preferably 22% by mass or less, still more preferably 20% by mass or less, based on a total amount of the non-aqueous electrolyte solution. By controlling the content of the other lithium salt to be in this range, the ionic conductivity can be increased appropriately.

In cases where the other lithium salt is used as an auxiliary salt, the content thereof may be set arbitrarily as long as the effects of the present invention A are not markedly impaired; however, it is preferably not less than 0.01% by mass, more preferably not less than 0.05% by mass, still more preferably not less than 0.1% by mass, based on a total amount of the non-aqueous electrolyte solution. Meanwhile, an upper limit value is preferably 20% by mass or less, more preferably 10% by mass or less, still more preferably 5% by mass or less, especially preferably 3% by mass or less, particularly preferably 2% by mass or less, based on a total amount of the non-aqueous electrolyte solution.

In the final composition of the non-aqueous electrolyte solution of the present invention A, the concentration of all of electrolytes such as the above-described lithium salts may be set arbitrarily as long as the effects of the present invention A are not markedly impaired; however, it is preferably 0.5 mol/L or higher, more preferably 0.6 mol/L or higher, still more preferably 0.7 mol/L or higher, but preferably 3 mol/L or lower, more preferably 2 mol/L or lower, still more preferably 1.8 mol/L or lower. The content of all of electrolytes in terms of % by mass is preferably not less than 6% by mass, more preferably not less than 7% by mass, still more preferably not less than 8% by mass, but preferably 30% by mass or less, more preferably 22% by mass or less, still more preferably 20% by mass or less, based on a total amount of the non-aqueous electrolyte solution. By controlling the content of the lithium salts to be in this range, the ionic conductivity can be increased appropriately.

The above-exemplified other lithium salts are identified and the content thereof is measured by ion chromatography.

In cases where the non-aqueous electrolyte solution of the present invention contains at least one other lithium salt selected from LiPF₆, LiBF₄, LiClO₄, and Li(CF₃SO₂)₂N as a main electrolyte, the mass ratio of the content of the heteroatom-containing lithium salt selected from the group consisting of (A) a lithium salt having an F-S bond, (B) a lithium salt having an oxalic acid skeleton, and (C) a lithium salt having P=O and P-F bonds with respect to the content of the other lithium salt in the non-aqueous electrolyte solution (heteroatom-containing lithium salt (% by mass)/other lithium salt (% by mass)) is not particularly restricted as long as the effects of the present invention are not markedly impaired; however, it is preferably 0.002 or higher, more preferably 0.02 or higher, particularly preferably 0.04 or higher. Meanwhile, an upper limit value is preferably 0.8 or lower, more preferably 0.5 or lower, particularly preferably 0.2 or lower. When the mass ratio of the above-described compounds is in this preferred range, an effect of improving the durability and the charging characteristics is more likely to be exerted without deterioration of other battery performance.

### <A5. Additives>

The non-aqueous electrolyte solution of the present invention A may further contain various additives within a range that does not markedly impair the effects of the present invention A, in addition to the above-described compounds. Examples of the additives include: cyano group-containing compounds, such as malononitrile, succinonitrile, glutaronitrile, adiponitrile, pimelonitrile, suberonitrile, azelanitrile, sebaconitrile, undecanedinitrile, and dodecanedinitrile; isocyanate compounds, such as 1,3-bis(isocyanatomethyl)cyclohexane, 1,4-bis(isocyanatomethyl)cyclohexane, 1,3-phenylene diisocyanate, 1,4-phenylene diisocyanate, 1,2-bis(isocyanatomethyl)benzene, 1,3-bis(isocyanatomethyl)benzene, and 1,4-bis(isocyanatomethyl)benzene; carboxylic anhydride compounds, such as acrylic anhydride, 2-methylacrylic anhydride, 3-methylacrylic anhydride, benzoic anhydride, 2-methylbenzoic anhydride, 4-methylbenzoic anhydride, 4-*tert*-butylbenzoic anhydride, 4-fluorobenzoic anhydride, 2,3,4,5,6-pentafluorobenzoic anhydride, methoxyformic anhydride, ethoxyformic anhydride, succinic anhydride, and maleic anhydride; thioether compounds, such as trimethyl[2-(phenylthio)ethoxy]silane, trimethyl[1-fluoro-2-(phenylthio)ethoxy]silane, and trimethyl[2-fluoro-2-(phenylthio)ethoxy]silane; cyclic carbonates having an unsaturated bond, such as vinylene carbonate and vinylethylene carbonate; fluorine atom-containing carbonates, such as fluoroethylene carbonate; sulfonic acid ester compounds, such as 1,3-propane sultone; and overcharge inhibitors, such as cyclohexylbenzene, *t*-butylbenzene, *t*-amylbenzene, biphenyl, alkylbiphenyls, terphenyl, partially hydrogenated terphenyl, diphenyl ether, and dibenzofuran. These compounds may be used in combination as appropriate. Among these compounds, from the standpoint of the capacity retention rate, vinylene carbonate or fluoroethylene carbonate is particularly preferred, and it is more preferred to use these carbonates in combination.

The non-aqueous electrolyte solution according to one embodiment of the present invention B will now be described in detail. The following descriptions are merely examples (representative examples) of the present invention B, and the present invention B is not limited thereto. Further, the present invention B can be carried out with any modification within the gist of the present invention.

### [Non-aqueous Electrolyte Solution B]

The non-aqueous electrolyte solution according to one embodiment of the present invention B contains: a non-aqueous solvent; a lithium salt as an electrolyte; and a compound represented by Formula (2) (hereinafter, may be referred as "compound (2)"), and at least one of a compound represented by Formula (3) (hereinafter, may be referred as "compound (3)") and an isocyanate compound (hereinafter, may be referred as "compound (4)"): 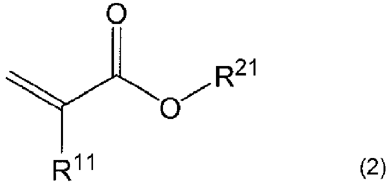 (wherein, R¹¹ represents a hydrogen atom or a methyl group, and R²¹ represents a fluorine atom-containing hydrocarbon group having 1 to 10 carbon atoms) 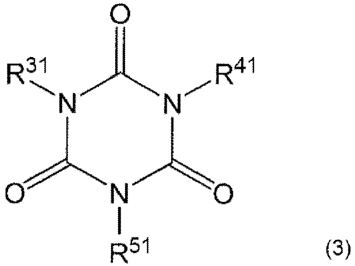 (wherein, R³¹ to R⁵¹ may be the same or different from each other, and each represent an optionally substituted organic group having 1 to 20 carbon atoms).

The non-aqueous electrolyte solution of the present invention B has an effect of exerting both excellent durability and excellent charging characteristics. The reason why the present invention B has this effect is not clear; however, it is presumed that the effect is attributed to the following mechanism. The acryloyl group or the methacryloyl group contained in the compound (2) as a partial structure undergoes a polymerization reaction with an anion species generated in the vicinity of a negative electrode, and an underlayer to which fluorine atom-containing carboxylic acid ester groups are bound is thereby formed on the negative electrode. After the formation of this underlayer, a specific nitrogen atom-containing compound (Compound (3) or Compound (4)) and a lithium salt contained in the system as an electrolyte react with each other to form a coating film containing lithium atoms and nitrogen atoms on the underlayer. In this process, since the coating film containing lithium is formed on the underlayer containing a fluorine atom-containing carboxylic acid ester, fluorine of the underlayer and lithium of the coating film react with each other to form a coating film containing a large amount of lithium fluoride. This coating film containing a large amount of lithium fluoride is known to have a high durability, and is thus believed to provide excellent durability. In addition, it is believed that, since the coating film contains nitrogen atoms originated from the nitrogen atom-containing compound, the mobility of lithium ions inside the coating film is enhanced, so that the charging characteristics are improved. In other words, it is presumed that a compound having a fluorine atom and a polymerizable group forms an underlayer, and a nitrogen atom-containing specific compound and a lithium salt as an electrolyte form a coating film on the underlayer, as a result of which the underlayer and the coating film react with each other to synergistically form a favorable coating film that has both satisfactory durability and satisfactory charging characteristics.

### <B1. Compound (2) Represented by Formula (2)>

The non-aqueous electrolyte solution of the present invention B contains a compound (2) represented by Formula (2). In Formula (2), R¹¹ represents a hydrogen atom or a methyl group, and R²¹ represents a fluorine atom-containing hydrocarbon group having 1 to 10 carbon atoms.

R²¹ is preferably a fluorine atom-containing hydrocarbon group having 1 to 5 carbon atoms, more preferably a fluorine atom-containing hydrocarbon group having 2 carbon atoms such as a trifluoroethyl group, a fluorine atom-containing hydrocarbon group having 3 carbon atoms such as a hexafluoroisopropyl group, or a fluorine atom-containing hydrocarbon group having 4 carbon atoms such as a hexafluorobutyl group, particularly preferably a trifluoroethyl group or a hexafluoroisopropyl group.

Specific examples of the compound (2) include 2,2,2-trifluoroethyl acrylate, 2,2,2-trifluoroethyl methacrylate, 1,1,1,3,3,3-hexafluoroisopropyl acrylate, and 1,1,1,3,3,3-hexafluoroisopropyl methacrylate. Thereamong, 2,2,2-trifluoroethyl acrylate and 2,2,2-trifluoroethyl methacrylate are preferred since they have an optimum reaction potential.

The content of the compound (2) in the non-aqueous electrolyte solution is not particularly restricted as long as the effects of the present invention B are not markedly impaired. Specifically, a lower limit value of the content of the compound (2) in the non-aqueous electrolyte solution is preferably not less than 0.001% by mass, more preferably not less than 0.05% by mass, still more preferably not less than 0.1% by mass. Meanwhile, an upper limit value is preferably 20% by mass or less, more preferably 10% by mass or less, still more preferably 5% by mass or less, particularly preferably 2% by mass or less. When the concentration of the compound (2) is in the above-described preferred range, an effect of improving the durability and the charging characteristics is more likely to be exerted without deterioration of other battery performance. A method for identifying the compound (2) and measuring the content thereof is not particularly restricted, and any known method may be selected as appropriate. Examples thereof include nuclear magnetic resonance (NMR) spectroscopy and gas chromatography.

### <B2. Specific Nitrogen-Containing Compound>

The non-aqueous electrolyte solution of the present invention B contains at least one of a compound represented by Formula (3) and an isocyanate compound.

### <B2-1. Compound (3) Represented by Formula (3)>

The compound (3) is a compound represented by the following Formula (3):

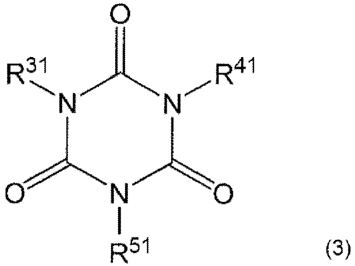

In Formula (3), R³¹ to R⁵¹ may be the same or different from each other, and each represent an optionally substituted organic group having 1 to 20 carbon atoms. The term "organic group" used herein refers to a functional group constituted by atoms selected from the group consisting of a carbon atom, a hydrogen atom, a nitrogen atom, an oxygen atom, and a halogen atom. Specific examples of the organic group include an alkyl group, an alkenyl group, an alkynyl group, an aryl group, an alkoxy group, a nitrile group, an ether group, a carbonate group, and a carbonyl group. R³¹ to R⁵¹ are each preferably a group having an unsaturated carbon-carbon bond, such as a vinyl group, an allyl group, an ethinyl group, a propargyl group, an acryl group via an alkyl group, a methacryl group via an alkyl group, a vinylsulfonyl group via an alkyl group, a fluorine-substituted vinyl group, and a fluorine-substituted allyl group; particularly preferably a vinyl group optionally substituted with fluorine, an allyl group optionally substituted fluorine, an acryl group via an alkyl group, a methacryl group via an alkyl group, or a vinylsulfonyl group via an alkyl group; more preferably an allyl group. From the standpoint of symmetry, R³¹ to R⁵¹ are preferably the same.

The content of the compound (3) in the non-aqueous electrolyte solution of the present invention B is not restricted and may be set arbitrarily as long as the effects of the present invention B are not markedly impaired; however, the compound (3) is contained in an amount of usually not less than 0.001% by mass, preferably not less than 0.01% by mass, more preferably not less than 0.1% by mass, but usually 10% by mass or less, preferably 5% by mass or less, more preferably 3% by mass or less, still more preferably 2% by mass or less, particularly preferably 1% by mass or less, with respect to a total amount of the non-aqueous electrolyte solution. A method for identifying the compound (3) and measuring the content thereof is not particularly restricted, and any known method may be selected as appropriate. Examples thereof include nuclear magnetic resonance (NMR) spectroscopy and gas chromatography.

When the concentration of the compound (3) is in the above-described preferred range, an effect of improving the durability and the charging characteristics is more likely to be exerted without deterioration of other battery performance.

### <B2-2. Compound (4)>

The isocyanate compound (compound (4)) is not particularly restricted in terms of its type as long as it is a compound that contains an isocyanate group in the molecule.

Specific examples of the compound (4) include:
aliphatic hydrocarbon monoisocyanate compounds, such as methyl isocyanate, ethyl isocyanate, cyclohexyl isocyanate, vinyl isocyanate, and allyl isocyanate;
aliphatic hydrocarbon diisocyanate compounds, for example, linear aliphatic hydrocarbon diisocyanates such as butyl diisocyanate and hexamethylene diisocyanate, and alicyclic hydrocarbon diisocyanates such as 1,3-bis(isocyanatomethyl)cyclohexane and dicyclohexylmethane-4,4'-diisocyanate;
aromatic hydrocarbon monoisocyanate compounds, for example, aromatic monoisocyanates such as phenyl isocyanate and (*ortho-*, *meta-*, or *para*-)toluene isocyanate, and aromatic monosulfonyl isocyanates such as (*ortho-*, *meta-*, or *para*-)toluene sulfonyl isocyanate; and
aromatic hydrocarbon diisocyanate compounds, such as *m-*xylylene diisocyanate, tolylene-2,4-diisocyanate, and diphenylmethane diisocyanate.

The compound (4) is preferably an aliphatic hydrocarbon diisocyanate compound, such as a linear aliphatic hydrocarbon diisocyanate or an alicyclic hydrocarbon diisocyanate; an aromatic hydrocarbon monoisocyanate compound, such as an aromatic monoisocyanate or an aromatic monosulfonyl isocyanate; or an aromatic hydrocarbon diisocyanate compound.

The compound (4) is more preferably a linear aliphatic hydrocarbon diisocyanate such as hexamethylene diisocyanate, or an alicyclic hydrocarbon isocyanate compound such as 1,3-bis(isocyanatomethyl)cyclohexane, particularly preferably hexamethylene diisocyanate or 1,3-bis(isocyanatomethyl)cyclohexane.

The content of the isocyanate compound in the non-aqueous electrolyte solution of the present invention B is not restricted and may be set arbitrarily as long as the effects of the present invention B are not markedly impaired; however, the isocyanate compound is contained in an amount of usually not less than 0.001% by mass, preferably not less than 0.01% by mass, more preferably not less than 0.1% by mass, but usually 10% by mass or less, preferably 5% by mass or less, more preferably 3% by mass or less, still more preferably 2% by mass or less, particularly preferably 1% by mass or less, with respect to a total amount of the non-aqueous electrolyte solution. A method for identifying the isocyanate compound and measuring the content thereof is not particularly restricted, and any known method may be selected as appropriate. Examples thereof include nuclear magnetic resonance (NMR) spectroscopy and gas chromatography.

A compound selected from the group consisting of the compound represented by Formula (3) and the isocyanate compound may be used singly, or two or more thereof may be used in any combination at any ratio.

### <B3. Non-aqueous Solvent>

Similarly to a general non-aqueous electrolyte solution, the non-aqueous electrolyte solution of the present invention B usually contains, as its main component, a non-aqueous solvent that dissolves the electrolytes described below. The non-aqueous solvent is not particularly restricted, and any known organic solvent can be used. The organic solvent can be the same as in the invention A. In other words, the organic solvent is not particularly restricted; however, it is preferably, for example, at least one selected from a saturated cyclic carbonate, a linear carbonate, a linear carboxylic acid ester, a cyclic carboxylic acid ester, an ether-based compound, and a sulfone-based compound. Specific examples of these organic solvents include the same ones as those exemplified above for the invention A, and their preferred modes are also the same as in the invention A. These non-aqueous solvents may be used singly, or in combination of two or more thereof.

<B4. Electrolytes>

The non-aqueous electrolyte solution of the present invention B usually contains a lithium salt as an electrolyte.

Examples of the lithium salt used in the present invention B include LiClO₄, LiBF₄, LiPF₆, LiAsF₆, LiTaF₆, LiCF₃SO₃, LiC₄F₉SO₃, Li(FSO₂)₂N, Li(CF₃SO₂)₂N, Li(C₂F₅SO₂)₂N, Li(CF₃SO₂)₃C, LiBF₃(C₂F₅), LiB(C₂O₄)₂, LiB(C₆F₅)₄, and LiPF₃(C₂F₅)₃. Thereamong, the lithium salt is preferably LiPF₆, LiBF₄, LiClO₄, LiB(C₂O₄)₂, Li(FSO₂)₂N, or Li(CF₃SO₂)₂N, more preferably LiPF₆, LiBF₄, Li(FSO₂)₂N, or Li(CF₃SO₂)₂N, still more preferably at least either one of LiPF₆ and Li(FSO₂)₂N, particularly preferably LiPF₆. The above-exemplified lithium salts may be used singly, or in combination of two or more thereof.

In the final composition of the non-aqueous electrolyte solution of the present invention B, the concentration of an electrolyte such as the lithium salt may be set arbitrarily as long as the effects of the present invention are not markedly impaired; however, it is preferably 0.5 mol/L or higher, more preferably 0.6 mol/L or higher, still more preferably 0.7 mol/L or higher, but preferably 3 mol/L or lower, more preferably 2 mol/L or lower, still more preferably 1.8 mol/L or lower. The content (% by mass) of an electrolyte is preferably not less than 6% by mass, more preferably not less than 7% by mass, still more preferably not less than 8% by mass, but preferably 30% by mass or less, more preferably 22% by mass or less, still more preferably 20% by mass or less, based on a total amount of the non-aqueous electrolyte solution. By controlling the concentration of an electrolyte to be in this range, the ionic conductivity can be increased appropriately. In cases where two or more electrolytes are used in combination, the ratio of each electrolyte may be set arbitrarily.

### <B5. Additives>

The non-aqueous electrolyte solution of the present invention B may further contain various additives within a range that does not markedly impair the effects of the present invention B, in addition to the above-described compounds. Examples of the additives include: cyano group-containing compounds, such as malononitrile, succinonitrile, glutaronitrile, adiponitrile, pimelonitrile, suberonitrile, azelanitrile, sebaconitrile, undecanedinitrile, and dodecanedinitrile; carboxylic anhydride compounds, such as acrylic anhydride, 2-methylacrylic anhydride, 3-methylacrylic anhydride, benzoic anhydride, 2-methylbenzoic anhydride, 4-methylbenzoic anhydride, 4-*tert*-butylbenzoic anhydride, 4-fluorobenzoic anhydride, 2,3,4,5,6-pentafluorobenzoic anhydride, methoxyformic anhydride, ethoxyformic anhydride, succinic anhydride, and maleic anhydride; thioether compounds, such as trimethyl[2-(phenylthio)ethoxy]silane, trimethyl[1-fluoro-2-(phenylthio)ethoxy]silane, and trimethyl[2-fluoro-2-(phenylthio)ethoxy]silane; cyclic carbonates having an unsaturated bond, such as vinylene carbonate and vinylethylene carbonate; fluorine atom-containing carbonates, such as fluoroethylene carbonate; sulfonic acid ester compounds, such as 1,3-propane sultone; phosphates, such as lithium difluorophosphate; and sulfonates, such as lithium fluorosulfonate. It is noted here that the above-exemplified lithium difluorophosphate and lithium fluorosulfonate each correspond to a lithium salt; however, hereinafter, they are not handled as electrolytes but rather regarded as additives from the standpoint of the degree of ionization in the non-aqueous solvent used in the non-aqueous electrolyte solution. Further, examples of an overcharge inhibitor include cyclohexylbenzene, t-butylbenzene, t-amylbenzene, biphenyl, alkylbiphenyls, terphenyl, partially hydrogenated terphenyl, diphenyl ether, and dibenzofuran. These compounds may be used in combination as appropriate. Among these compounds, from the standpoint of the capacity retention rate, vinylene carbonate, fluoroethylene carbonate, lithium difluorophosphate, or lithium fluorosulfonate is particularly preferred, and it is more preferred to use these compounds in combination.

### [Non-aqueous Electrolyte Battery]

A non-aqueous electrolyte secondary battery (hereinafter, may be referred to as "the non-aqueous electrolyte secondary battery of the present invention") can be obtained using the non-aqueous electrolyte solution of the present invention A or the non-aqueous electrolyte solution of the present invention B, along with a positive electrode and a negative electrode.

The non-aqueous electrolyte secondary battery according to one embodiment of the present invention A is a non-aqueous electrolyte secondary battery including: a negative electrode and a positive electrode that are capable of occluding and releasing metal ions; and a non-aqeuous electrolyte solution, and this non-aqueous electrolyte solution is the non-aqueous electrolyte solution of the present invention A.

The non-aqueous electrolyte secondary battery according to one embodiment of the present invention B is a non-aqueous electrolyte secondary battery including: a negative electrode and a positive electrode that are capable of occluding and releasing metal ions; and a non-aqeuous electrolyte solution, and this non-aqueous electrolyte solution is the non-aqueous electrolyte solution of the present invention B.

Examples of the non-aqueous electrolyte secondary battery include lithium ion secondary batteries and sodium ion secondary batteries, and the non-aqueous electrolyte secondary battery is preferably a lithium ion secondary battery. The lithium ion secondary battery usually includes: the non-aqueous electrolyte solution of the present invention A or the non-aqueous electrolyte solution of the present invention B; a positive electrode, which includes a current collector and a positive electrode active material layer arranged on the current collector and is capable of occluding and releasing lithium ions; and a negative electrode, which includes a current collector and a negative electrode active material layer arranged on the current collector and is capable of occluding and releasing lithium ions.

### <1. Positive Electrode>

The positive electrode usually has a positive electrode active material layer on a current collector, and this positive electrode active material layer contains a positive electrode active material.

In the non-aqueous secondary battery of the present invention, examples of a positive electrode material that may be used as an active material of the positive electrode include: lithium-transition metal composite oxides, such as lithium-cobalt composite oxide having a basic composition represented by LiCoO₂, lithium-nickel composite oxide represented by LiNiO₂, and lithium-manganese composite oxide represented by LiMnO₂ or LiMn₂O₄; transition metal oxides, such as manganese dioxide; and mixtures of these composite oxides. Further, TiS₂, FeS₂, Nb₃S₄, Mo₃S₄, CoS₂, V₂O₅, CrO₃, V₃O₃, FeO₂, GeO₂, Li(Ni_{1/3}Mn_{1/3}Co_{1/3})O₂, LiFePO₄ and the like may be used and, from the standpoint of the capacity density, for example, Li(Ni_{1/3}Mn_{1/3}Co_{1/3})O₂, Li(Ni_{0.5}Mn_{0.3}Co_{0.2})O₂, Li(Ni_{0.5}Mn_{0.2}Co_{0.3})O₂, Li(Ni_{0.6}Mn_{0.2}Co_{0.2})O₂, Li(Ni_{0.8}Mn_{0.1}Co_{0.1})O₂, and Li(Ni_{0.8}Co_{0.15}Al_{0.05})O₂ are particularly preferred.

### <2. Negative Electrode>

The negative electrode usually has a negative electrode active material layer on a current collector, and this negative electrode active material layer contains a negative electrode active material. The negative electrode active material will now be described.

The negative electrode active material is not particularly restricted as long as it is capable of electrochemically occluding and releasing s-block metal ions, such as lithium ions, sodium ions, potassium ions, and magnesium ions. Specific examples of the negative electrode active material include carbonaceous materials and metal compound-based materials, as well as oxides, carbides, nitrides, silicides, sulfides, and phosphides thereof. Any of these materials may be used singly, or two or more thereof may be used in any combination.

A carbonaceous material to be used as the negative electrode active material is not particularly restricted, and it is, for example, a graphite, an amorphous carbon, or a carbonaceous material having a low graphitization degree. Examples of the type of the graphite include natural graphites and artificial graphites. These graphites coated with a carbonaceous material, such as an amorphous carbon or a graphitized material, may be used as well. Examples of the amorphous carbon include particles obtained by firing a bulk mesophase, and particles obtained by infusibilizing and firing a carbon precursor. Examples of carbonaceous material particles having a low graphitization degree include those obtained by firing an organic substance usually at a temperature of lower than 2,500°C. Any of these materials may be used singly, or two or more thereof may be used in any combination. It is also preferred to use a carbonaceous material and Si in combination as the negative electrode active material.

A metal compound-based material to be used as the negative electrode active material is not particularly restricted, and examples thereof include compounds containing a metal or metalloid of Ag, Al, Bi, Cu, Ga, Ge, In, Ni, Pb, Sb, Si, Sn, Sr, Zn or the like. Thereamong, simple metals, alloys, oxides, carbides, nitrides and the like of silicon (Si) and tin (Sn) are preferred and, from the standpoints of the capacity per unit mass and the environmental load, simple Si and SiOx (wherein, 0.5 ≤ O ≤ 1.6) are particularly preferred.

### <3. Separator>

A separator is usually arranged between the positive electrode and the negative electrode for the purpose of inhibiting a short circuit. In this case, the separator is usually impregnated with the non-aqueous electrolyte solution.

The material and the shape of the separator are not particularly restricted, and any known material and shape can be employed as long as the separator does not markedly impair the effects of the present invention.

The material of the separator is not particularly restricted as long as it is a material stable against the non-aqueous electrolyte solution, for example, resins such as polyolefins (e.g., polyethylenes and polypropylenes), polytetrafluoroethylenes, and polyether sulfones; oxides, such as alumina and silicon dioxide; nitrides, such as aluminum nitride and silicon nitride; sulfates, such as barium sulfate and calcium sulfate; and glass filters composed of glass fibers can be used. Thereamong, glass filters and polyolefins are preferred, and polyolefins are more preferred. Any of these materials may be used singly, or two or more thereof may be used in any combination at any ratio. The above-described materials may be laminated as well.

The separator may have any thickness; however, the thickness is usually 1 µm or greater, preferably 5 µm or greater, more preferably 10 µm or greater, but usually 50 µm or less, preferably 40 µm or less, more preferably 30 µm or less. When the separator is overly thinner than this range, the insulation and the mechanical strength may be reduced. Meanwhile, when the separator is overly thicker than this range, not only the battery performance such as rate characteristics may be deteriorated, but also the energy density of the non-aqueous electrolyte secondary battery as a whole may be reduced.

Examples of the form of the separator include a nonwoven fabric, a woven fabric, and a thin film such as a microporous film. As a thin-film separator, one having a pore size of 0.01 to 1 µm and a thickness of 5 to 50 µm is preferably used. Aside from such an independent thin-film separator, a separator obtained by forming a composite porous layer that contains particles of an inorganic material on the surface layer of the positive electrode and/or that of the negative electrode using a resin binder can be used as well. For example, on both sides of the positive electrode, a porous layer may be formed using alumina particles having a 90% particle size of smaller than 1 µm along with a fluorine resin as a binder.

The separator is preferably in the form of a microporous film or a nonwoven fabric that has excellent liquid retainability. In cases where a porous separator in the form of a porous sheet, a nonwoven fabric or the like is used, the porosity of the separator may be set arbitrarily; however, it is usually 20% or higher, preferably 35% or higher, more preferably 45% or higher, but usually 90% or lower, preferably 85% or lower, more preferably 75% or lower. When the porosity is overly lower than this range, the membrane resistance is increased, and this tends to deteriorate the rate characteristics. Meanwhile, when the porosity is overly higher than this range, the mechanical strength and the insulation of the separator tend to be reduced.

The average pore size of the separator may also be set arbitrarily; however, it is usually 0.5 µm or smaller, preferably 0.2 µm or smaller, but usually 0.05 µm or larger. When the average pore size is larger than this range, a short circuit is likely to occur. Meanwhile, when the average pore size is smaller than this range, the membrane resistance is increased, and this may lead to deterioration of the rate characteristics.

### <4. Conductive Material>

The positive electrode and the negative electrode may contain a conductive material for improvement of the electrical conductivity. As the conductive material, any known conductive material can be used. Specific examples thereof include: metal materials, such as copper and nickel; and carbonaceous materials, for example, graphites such as natural graphites and artificial graphites, carbon blacks such as acetylene black, and amorphous carbon such as needle coke. Any of these conductive materials may be used singly, or two or more thereof may be used in any combination at any ratio.

The conductive material is used such that it is incorporated in an amount of usually not less than 0.01 parts by mass, preferably not less than 0.1 parts by mass, more preferably not less than 1 part by mass, but usually 50 parts by mass or less, preferably 30 parts by mass or less, more preferably 15 parts by mass or less, with respect to 100 parts by mass of the positive electrode material or the negative electrode material. When the content of the conductive material is lower than this range, the electrical conductivity may be insufficient. Meanwhile, when the content of the conductive material is higher than this range, the battery capacity may be reduced. In the present specification, the positive electrode material is a positive electrode mixture that contains a positive electrode active material, a conductive material, a binder, and the like. The negative electrode material is a negative electrode mixture that contains a negative electrode active material, a binder, a thickening agent, and the like.

### <5. Binder>

The positive electrode and the negative electrode may contain a binder for improvement of the bindability. The binder is not particularly restricted as long as it is a material that is stable against the non-aqueous electrolyte solution and the solvent used in the electrode production.

When a coating method is employed, the binder may be any material that can be dissolved or dispersed in a liquid medium used in the electrode production, and specific examples of such a binder include: resin-based polymers, such as polyethylene, polypropylene, polyethylene terephthalate, polymethyl methacrylate, aromatic polyamides, cellulose, and nitrocellulose; rubbery polymers, such as SBR (styrene-butadiene rubbers), NBR (acrylonitrile-butadiene rubbers), fluororubbers, isoprene rubbers, butadiene rubbers, and ethylene-propylene rubbers; thermoplastic elastomeric polymers, such as styrene-butadiene-styrene block copolymers and hydrogenation products thereof, EPDM (ethylene-propylene-diene terpolymers), styrene-ethylene-butadiene-ethylene copolymers, and styrene-isoprene-styrene block copolymers and hydrogenation products thereof; soft resinous polymers, such as syndiotactic 1,2-polybutadiene, polyvinyl acetate, ethylene-vinyl acetate copolymers, and propylene-*α*-olefin copolymers; fluorine-based polymers, such as polyvinylidene fluoride (PVdF), polytetrafluoroethylene, and tetrafluoroethylene-ethylene copolymers; and polymer compositions having ionic conductivity for alkali metal ions (particularly lithium ions). Any of these substances may be used singly, or two or more thereof may be used in any combination at any ratio.

The ratio of the binder is usually 0.1 parts by mass or higher, preferably 1 part by mass or higher, more preferably 3 parts by mass or higher, but usually 50 parts by mass or lower, preferably 30 parts by mass or lower, more preferably 10 parts by mass or lower, still more preferably 8 parts by mass or lower, with respect to 100 parts by mass of the positive electrode material or the negative electrode material. When the ratio of the binder is in this range, the bindability of the respective electrodes can be sufficiently maintained, so that the mechanical strength of the electrodes can be ensured, which is preferred from the standpoints of the cycle characteristics, the battery capacity, and the electrical conductivity.

### <6. Liquid Medium>

The type of a liquid medium used for the formation of a slurry is not particularly restricted as long as it is a solvent that is capable of dissolving or dispersing the active materials, the conductive material and the binder as well as a thickening agent used as required, and either an aqueous solvent or an organic solvent may be used.

Examples of the aqueous solvent include water, and mixed media of alcohol and water. Examples of the organic solvent include: aliphatic hydrocarbons, such as hexane; aromatic hydrocarbons, such as benzene, toluene, xylene, and methylnaphthalene; heterocyclic compounds, such as quinoline and pyridine; ketones, such as acetone, methyl ethyl ketone, and cyclohexanone; esters, such as methyl acetate and methyl acrylate; amines, such as diethylenetriamine and *N,N-*dimethylaminopropylamine; ethers, such as diethyl ether and tetrahydrofuran (THF); amides, such as N-methylpyrrolidone (NMP), dimethylformamide, and dimethylacetamide; and aprotic polar solvents, such as hexamethylphosphoramide and dimethyl sulfoxide. Any of these liquid media may be used singly, or two or more thereof may be used in any combination at any ratio.

<7. Thickening Agent>

When an aqueous medium is used as the liquid medium for the formation of a slurry, it is preferred to prepare the slurry using a thickening agent and a latex such as a styrene-butadiene rubber (SBR). The thickening agent is usually used for the purpose of adjusting the viscosity of the resulting slurry.

The thickening agent is not restricted as long as it does not markedly limit the effects of the present invention, and specific examples of the thickening agent include carboxymethyl cellulose, methyl cellulose, hydroxymethyl cellulose, ethyl cellulose, polyvinyl alcohol, oxidized starch, phosphorylated starch, casein, and salts thereof. Any of these thickening agents may be used singly, or two or more thereof may be used in any combination at any ratio.

In cases where a thickening agent is used, it is desired that the amount thereof be usually not less than 0.1 parts by mass, preferably not less than 0.5 parts by mass, more preferably not less than 0.6 parts by mass, but usually 5 parts by mass or less, preferably 3 parts by mass or less, more preferably 2 parts by mass or less, with respect to 100 parts by mass of the positive electrode material or the negative electrode material. When the amount of the thickening agent is less than this range, the coatability may be markedly reduced, while when the amount of the thickening agent is greater than this range, a reduction in the ratio of an active material in an active material layer may cause a reduction in the battery capacity and an increase in the resistance between the active materials.

### <8. Current Collector>

The material of the current collector is not particularly restricted, and any known material can be used. Specific examples thereof include: metal materials, such as aluminum, stainless steel, nickel-plated steel, titanium, tantalum, and copper; and carbonaceous materials, such as a carbon cloth and a carbon paper. Thereamong, a metal material, particularly aluminum, is preferred.

When the current collector is a metal material, the current collector may have any shape of, for example, a metal foil, a metal cylinder, a metal coil, a metal sheet, a metal thin film, an expanded metal, a punched metal, and a foamed metal and, when the current collector is a carbonaceous material, examples thereof include a carbon sheet, a carbon thin film, and a carbon cylinder. Thereamong, the current collector is preferably a metal thin film. The current collector may be in the form of a mesh as appropriate.

The current collector may have any thickness; however, the thickness is usually 1 µm or greater, preferably 3 µm or greater, more preferably 5 µm or greater, but usually 1 mm or less, preferably 100 µm or less, more preferably 50 µm or less. When the thickness of the thin film is in this range, a sufficient strength required for a current collector is maintained, and this is also preferred from the standpoint of the ease of handling.

### <9. Battery Design>

### [Electrode Group]

An electrode group may have either a layered structure in which the above-described positive electrode (hereinafter, also referred to as "positive electrode plate") and negative electrode (hereinafter, also referred to as "negative electrode plate") are layered with the above-described separator being interposed therebetween, or a wound structure in which the above-described positive electrode plate and negative electrode plate are spirally wound with the above-described separator being interposed therebetween. The volume ratio of the electrode group with respect to the internal volume of the battery (this volume ratio is hereinafter referred to as "electrode group occupancy") is usually 40% or higher, preferably 50% or higher, but usually 90% or lower, preferably 80% or lower. When the electrode group occupancy is lower than this range, the battery has a small capacity. Meanwhile, when the electrode group occupancy is higher than this range, since the void space is small, there are cases where an increase in the battery temperature causes swelling of members and an increase in the vapor pressure of an electrolyte liquid component, as a result of which the internal pressure is increased to deteriorate various properties of the battery, such as charge-discharge repeating performance and high-temperature storage characteristics, and to activate a gas release valve that relieves the internal pressure to the outside.

### [Current Collector Structure]

A current collector structure is not particularly restricted; however, in order to more effectively realize an improvement in the discharge characteristics attributed to the non-aqueous electrolyte solution of the present invention, it is preferred to adopt a structure that reduces the resistance of wiring and joint parts. By reducing the internal resistance in this manner, the effects of using the non-aqueous electrolyte solution of the present invention are particularly favorably exerted.

In an electrode group having the above-described layered structure, the metal core portions of the respective electrode layers are preferably bundled and welded to a terminal. When the area of one electrode is large, the internal resistance is high; therefore, it is also preferred to reduce the resistance by arranging plural terminals in each electrode. In an electrode group having the above-described wound structure, the internal resistance can be reduced by arranging plural lead structures on each of the positive electrode and the negative electrode and bundling them to a terminal.

### [Protective Element]

Examples of a protective element include: a PTC (Positive Temperature Coefficient) element, a thermal fuse, and a thermistor, whose resistance increases with heat generation caused by excessive current flow or the like; and a valve (current cutoff valve) that blocks an electric current flowing into a circuit in response to a rapid increase in the battery internal pressure or internal temperature in the event of abnormal heat generation. The protective element is preferably selected from those that are not activated during normal use at a high current, and it is more preferred to design the battery such that neither abnormal heat generation nor thermal runaway occurs even without a protective element from the standpoint of attaining a high output.

### [Outer Package]

The non-aqueous electrolyte secondary battery of the present invention is usually constructed by housing the above-described non-aqueous electrolyte solution, negative electrode, positive electrode, separator and the like in an outer package. This outer package is not restricted, and any known outer package can be employed as long as it does not markedly impair the effects of the present invention.

Specifically, the outer package is not particularly restricted as long as it is made of a substance that is stable against the non-aqueous electrolyte solution to be used. Usually, for example, a metal such as a nickel-plated steel sheet, stainless steel, aluminum or an aluminum alloy, nickel, titanium, or a magnesium alloy; or a layered film (laminated film) composed of a resin and an aluminum foil is used. From the standpoint of weight reduction, it is preferred to use a metal such as aluminum or an aluminum alloy, or a laminated film.

Examples of an outer casing using any of the above-described metals include those having a hermetically sealed structure obtained by welding metal pieces together by laser welding, resistance welding or ultrasonic welding, and those having a caulked structure obtained using the above-described metals via a resin gasket. Examples of an outer casing using the above-described laminated film include those having a hermetically sealed structure obtained by heat-fusing resin layers together. In order to improve the sealing performance, a resin different from the resin used in the laminated film may be interposed between the resin layers. Particularly, in the case of forming a sealed structure by heat-fusing resin layers via a collector terminal, since it involves bonding between a metal and a resin, a polar group-containing resin or a resin modified by introduction of a polar group is preferably used as the resin to be interposed.

### [2-4-5. Shape]

Further, the shape of the outer package may be selected arbitrarily, and the outer package may have any of, for example, a cylindrical shape, a prismatic shape, a laminated shape, a coin shape, and a large-sized shape.

### EXAMPLES

### [Experiment A]

The present invention A will now be described more concretely by way of Examples and Comparative Examples; however, the present invention A is not restricted to the below-described Examples within the gist of the present invention A.

### [Preparation of Non-aqueous Electrolyte Solutions]

### (Examples A1 to A14 and Comparative Examples A1 to A13)

An electrolyte solution was prepared by dissolving LiPF₆ at a ratio of 1 mol/L in a mixture of ethylene carbonate and ethyl methyl carbonate (volume ratio = 3:7), and this electrolyte solution was used as a basic electrolyte solution A. The compounds shown below were each added to this basic electrolyte solution in the respective amounts (% by mass) shown in Tables 1 to 4 to prepare electrolyte solutions. In the Tables below, "heteroatom-containing lithium salt (A)", "heteroatom-containing lithium salt (A)" and "heteroatom-containing lithium salt (C)" are indicated as "Lithium salt (A)", "Lithium salt (A)" and "Lithium salt (C)", respectively. In the Tables below, "Content (% by mass)" indicates the content of each compound, taking a total amount of the respective non-aqueous electrolyte solutions as 100% by mass.

### <Compounds>

### Compound 1-1: 2,2,2-trifluoroethyl acrylate

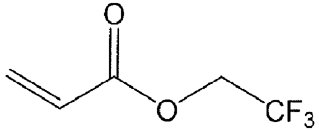

### Compound 1-2: 2,2,2-trifluoroethyl methacrylate

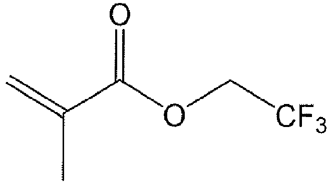

### Compound 1-3: 1,1,1,3,3,3-hexafluoroisopropyl acrylate

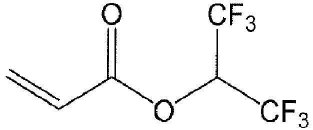

### Compound 1-4: 1,1,1,3,3,3-hexafluoroisopropyl methacrylate

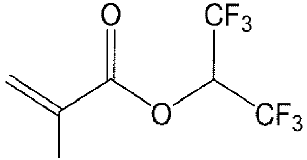

### Compound 2: 2,2,3,3-tetrafluoropropyl methacrylate

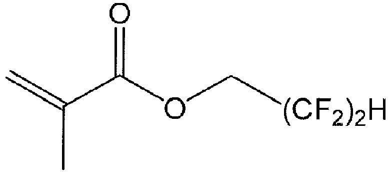

### Compound A-1: lithium fluorosulfonate

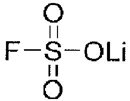

### Compound A-2: lithium bis(fluorosulfonyl)imide

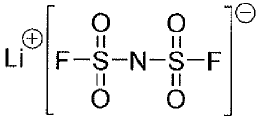

### Compound B: lithium bis(oxalato)borate

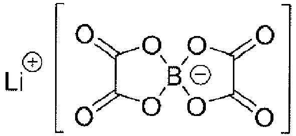

### Compound C: lithium difluorophosphate

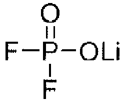

### Compound D: lithium tetrafluoroborate

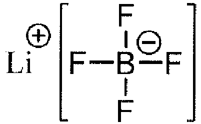

### [Production of Electrodes]

### (Examples A1 to A11 and Comparative Examples A1 to A13)

Using a mixer, 50 parts by mass of SiO as a negative electrode active material, 25 parts by mass of polyacrylic acid as a binder, and 25 parts by mass of carbon black as a conductive material were kneaded to prepare a slurry. The thus obtained slurry was applied and dried onto a 20 µm-thick copper foil by a blade method, and the resultant was roll-pressed using a press machine to prepare a negative electrode sheet. This negative electrode sheet was punched out in a disk shape of 12.5 mm in diameter to produce a negative electrode. In addition, a lithium metal foil was punched out in a disk shape of 14 mm in diameter to produce a counter electrode.

### (Examples A12 to A14)

Using a mixer, 3 parts by mass of Si and 94.5 parts by mass of a carbonaceous material as negative electrode active materials, 1.5 parts by mass of sodium carboxymethyl cellulose as a thickening agent, and 1 part by mass of a styrene-butadiene rubber as a binder were kneaded to prepare a slurry. The thus obtained slurry was applied and dried onto a 20 µm-thick copper foil by a blade method, and the resultant was roll-pressed using a press machine to prepare a negative electrode sheet. This negative electrode sheet was punched out in a disk shape of 12.5 mm in diameter to produce a negative electrode. In addition, a lithium metal foil was punched out in a disk shape of 14 mm in diameter to produce a counter electrode.

### [Production of Lithium Secondary Batteries]

### (Examples A1 to A14 and Comparative Examples A1 to A16)

Coin-type lithium ion secondary batteries were each produced by laminating the negative electrode produced above, a separator impregnated with each electrolyte solution prepared above, and the counter electrode produced above, and sealing the resultant in a coin-shaped metal container.

### [Charge-Discharge Measurement]

### (Examples A1 to A14 and Comparative Examples A1 to A16)

Each battery was charged and discharged four times at a current value of 1 C in a voltage range of 1.5 V to 5 mV at 25°C to be stabilized. Subsequently, the battery was charged to 5 mV at a current value of 0.1 C, further charged to a current density of 0.01 C with a constant voltage of 5 mV, and then discharged to 1,500 mV at a current value of 0.1 C. The discharge capacity in this process was defined as "reference capacity". Thereafter, 19 cycles, each of which consisted of charging the battery to 5 mV at 1 C, further charging the battery to a current density of 0.01 C with a constant voltage of 5 mV, and then discharging the battery to 1,500 mV at 1 C followed by evaluation, were performed as a high-rate test. Finally, an operation of charging the battery to 5 mV at a current value of 0.1 C, further charging the battery to a current density of 0.01 C at a constant voltage of 5 mV, and then discharging the battery to 1,500 mV at a current value of 0.1 C was performed, and the discharge capacity in this operation was defined as "discharge capacity after cycle test".

### [Evaluation of Durability]

### (Examples A1 to A14 and Comparative Examples A1 to A16)

The cycle capacity retention rate (%) was calculated using the following equation: [(Discharge capacity after cycle test)/(Reference capacity)] × 100. In Examples A2 to A11 and Comparative Examples A1 to A16, the calculated retention rate was normalized such that the retention rate of Example A1 was 100. In Examples A13 and A14, the calculated retention rate was normalized such that the retention rate of Example A12 was 100. The results thereof are shown in Tables 1 to 4. It is noted here that the lithium secondary batteries of Examples A12 to A14 were different from the lithium secondary battery of Example A1 in terms of the negative electrode active material, and the retention rate of Examples A12 to A14 was 90, 91, and 92, respectively, taking the retention rate of Example A1 as 100.

### [Evaluation of Charging Characteristics]

### (Examples A1 to A14 and Comparative Examples A1 to A16)

In the above-described charge-discharge measurement, the charge capacity in the 19th cycle of the high-rate test was defined as "high-rate charge capacity". It can be said that the higher this capacity, the superior the high-current charging characteristics. In Examples A2 to A11 and Comparative Examples A1 to A16, the measured high-rate charge capacity was normalized such that the high-rate charge capacity of Example A1 was 100. In Examples A13 and A14, the measured high-rate charge capacity was normalized such that the high-rate charge capacity of Example A12 was 100. Further, the time required for charging 50% of the reference capacity in the 19th cycle of the high-rate test was defined as "high-rate charging time". It can be said that the shorter this time, the superior the high-current charging characteristics. In Examples A2 to A11 and Comparative Examples A1 to A16, the measured high-rate charging time was normalized such that the high-rate charging time of Example A1 was 100. In Examples A13 and A14, the measured high-rate charging time was normalized such that the high-rate charge capacity of Example A12 was 100. The results thereof are shown in Tables 1 to 4. It is noted here that the lithium secondary batteries of Examples A12 to A14 were different from the lithium secondary battery of Example A1 in terms of the negative electrode active material, and the high-rate charging time of Examples A12 to A14 was 150, 149, and 147, respectively, taking the high-rate charging time of Example A1 as 100.

**Table 1**

| | Compound (1) | | Specific heteroatom-containing lithium salt | | | | | | Negative electrode active material | Effect | | |
|---|---|---|---|---|---|---|---|---|---|---|---|---|
| | | | Lithium salt (A) | | Lithium salt (B) | | Lithium salt (C) | | | | | |
| | Compound | Content (% by mass) | Compound | Content (% by mass) | Compound | Content (% by mass) | Compound | Content (% by mass) | | Retenti on rate | High-rate charge capacity | High-rate charging time |
| Example A1 | Compound 1-1 | 0.25 | Compound A-1 | 0.25 | - | - | - | - | SiO | 100 | 100 | 100 |
| Example A2 | Compound 1-1 | 0.25 | Compound A-2 | 0.25 | - | - | - | - | SiO | 93 | 90 | 182 |
| Example A3 | Compound 1-1 | 0.25 | - | - | Compound B | 0.25 | - | - | SiO | 92 | 89 | 113 |
| Example A4 | Compound 1-1 | 0.25 | - | - | - | - | Compound C | 0.25 | SiO | 93 | 78 | 118 |
| Example A5 | Compound 1-2 | 0.25 | Compound A-1 | 0.25 | - | - | - | - | SiO | 95 | 93 | 230 |
| Example A6 | Compound 1-3 | 0.25 | Compound A-1 | 0.25 | - | - | - | - | SiO | 98 | 94 | 116 |
| Example A7 | Compound 1-4 | 0.25 | Compound A-1 | 0.25 | - | - | - | - | SiO | 99 | 97 | 137 |
| Example A8 | Compound 1-1 | 0.25 | Compound A-1 | 2 | - | - | - | - | SiO | 95 | 86 | 142 |
| Example A9 | Compound 1-1 | 0.25 | - | - | - | - | Compound C | 1.5 | SiO | 102 | 97 | 159 |
| Example A10 | Compound 1-1 | 0.25 | Compound A-1 | 1 | Compound B | 1 | Compound C | 1 | SiO | 97 | 88 | 116 |
| Example A11 | Compound 1-1 | 0.25 | Compound A-1 | 1 | - | - | Compound C | 1 | SiO | 101 | 88 | 143 |
| Comparative Example A1 | Compound 1-1 | 0.25 | - | - | - | - | - | - | SiO | 85 | 74 | 376 |
| Comparative Example A2 | Compound 1-2 | 0.25 | - | - | - | - | - | - | SiO | 86 | 74 | 372 |
| Comparative Example A3 | Compound 1-3 | 0.25 | - | - | - | - | - | - | SiO | 79 | 66 | 446 |
| Comparative Example A4 | Compound 1-4 | 0.25 | - | - | - | - | - | - | SiO | 77 | 60 | 578 |
| Comparative Example A5 | - | - | Compound A-1 | 0.25 | - | - | - | - | SiO | 87 | 77 | 321 |
| Comparative Example A6 | - | - | Compound A-2 | 0.25 | - | - | - | - | SiO | 84 | 75 | 328 |
| Comparative Example A7 | - | - | - | - | Compound B | 0.25 | - | - | SiO | 81 | 71 | 397 |
| Comparative Example A8 | - | - | - | - | - | - | Compound C | 0.25 | SiO | 85 | 75 | 393 |
| Comparative Example A9 | Compound 1-1 | 0.25 | Compound A-1 | 6 | - | - | - | - | SiO | 77 | 72 | 699 |
| Comparative Example A10 | Compound 1-1 | 0.25 | - | - | Compound B | 6 | - | - | SiO | 76 | 47 | 83 |
| Comparative Example A11 | Compound 1-1 | 0.25 | - | - | - | - | Compound C | 6 | SiO | Not disolved, unmeasurable | | |

**Table 2**

| | Compound (1) | | Specific heteroatom-containing lithium salt | | | | | | Negative electrode active material | Effect | | |
|---|---|---|---|---|---|---|---|---|---|---|---|---|
| | | | Lithium salt (A) | | Lithium salt (B) | | Lithium salt (C) | | | | | |
| | Compound | Content (% by mass) | Compound | Content (% by mass) | Compound | Content (% by mass) | Compound | Content (% by mass) | | Retention rate | High-rate charge capacity | High-rate charging time |
| Example A12 | Compound 1-1 | 0.25 | Compound A-1 | 0.25 | - | - | - | - | Si/graphite | 100 | 100 | 100 |
| Example A13 | Compound 1-1 | 0.25 | - | - | Compound B | 0.25 | - | - | Si/graphite | 101 | 100 | 99 |
| Example A14 | Compound 1-1 | 0.25 | - | - | - | - | Compound C | 0.25 | Si/graphite | 102 | 96 | 98 |

**Table 3**

| | Fluorine-containi ng carboxylic acid ester compound other than compound (1) | | Specific heteroatom-containing lithium salt | | | | | | Effect | | |
|---|---|---|---|---|---|---|---|---|---|---|---|
| | | | Lithium salt (A) | | Lithium salt (B) | | Lithium salt (C) | | | | |
| | Compound | Content (% by mass) | Compound | Content (% by mass) | Compound | Content (% by mass) | Compound | Content (% by mass) | Retention rate | High-rate charge capacity | High-rate charging time |
| Comparative Example A12 | Compound A2 | 0.25 | Compound A-1 | 0.25 | - | - | - | - | 81 | 68 | 304 |
| Comparative Example A13 | Compound A2 | 0.25 | Compound A-2 | 0.25 | - | - | - | - | 80 | 59 | 525 |
| Comparative Example A14 | Compound A2 | 0.25 | - | - | Compound B | 0.25 | - | - | 77 | 60 | 415 |
| Comparative Example A15 | Compound A2 | 0.25 | - | - | - | - | Compound C | 0.25 | 82 | 61 | 672 |

**Table 4**

| | Compound (1) | | Lithium salt other than lithium salt (A)-(C) | | Effect | | |
|---|---|---|---|---|---|---|---|
| | Compound | Content (% by mass) | Compound | Content (% by mass) | Retention rate | High-rate charge capacity | High-rate charging time |
| Comparative Example A16 | Compound 1-1 | 0.25 | Compound D | 0.25 | 85 | 74 | 385 |

As apparent from Tables 1, 3 and 4 above, it is seen that the batteries produced in Examples A1 to A11 had higher retention rates, higher high-rate charge capacities, and shorter high-rate charging times than the batteries of Comparative Examples A1 to A16. These results indicate that both excellent durability and excellent charging characteristics are attained in a non-aqueous electrolyte secondary battery by using a non-aqueous electrolyte solution having a composition that includes a specific fluorine-containing carboxylic acid ester compound (compound (1)) and a specific heteroatom-containing lithium salt. As seen from Examples A1 to A4 and Comparative Examples A5 to A8 as well as Examples A1, A5, A6 and A7 and Comparative Examples A1 to A4, the compound (1) and specific heteroatom-containing lithium salt synergistically exert a favorable effect for the durability and the charging characteristics only when they both are incorporated. In addition, as seen from Examples A1 to A4 and Comparative Examples A12 to A15, among those fluorine-containing carboxylic acid ester compounds containing an acryloyl group or a methacryloyl group as a partial structure, only the ones corresponding to the compound (1) exert a favorable effect. Further, as seen from Example A1 and Comparative Example A16, a favorable effect is exerted only when a specific heteroatom-containing lithium salt is used. Moreover, from Examples A12 to A14, it is seen that, by using the non-aqueous electrolyte solution according to the present invention A, a non-aqueous electrolyte secondary battery having both excellent durability and excellent charging characteristics was obtained also when a carbonaceous material and Si were used in combination as negative electrode active materials. In the above-described Examples and Comparative Examples shown in Tables 1 to 4, the cycle test was conducted in a relatively short period as a model; however, significant differences were confirmed. The actual use of a non-aqueous electrolyte secondary battery may extend to several years; therefore, it is understandable that the above-described differences in the results would be more prominent, assuming the use over an extended period.

### [Experiment B]

The present invention B will now be described more concretely by way of Examples and Comparative Examples; however, the present invention B is not restricted to the below-described Examples within the gist of the present invention B.

### (Examples B1 to B9 and Comparative Examples B1 to B6)

### [Preparation of Non-aqueous Electrolyte Solutions]

An electrolyte solution was prepared by dissolving LiPF₆ at a ratio of 1 mol/L in a mixture of ethylene carbonate and ethyl methyl carbonate (volume ratio = 3:7), and this electrolyte solution was used as a basic electrolyte solution B. The compounds shown below were each added to this basic electrolyte solution B in the respective amounts (% by mass) shown in Table 5 to prepare electrolyte solutions. In Table 5, "Content (% by mass)" indicates the content of each compound, taking a total amount of the respective non-aqueous electrolyte solutions as 100% by mass.

### <Compounds>

### Compound B1-1: 2,2,2-trifluoroethyl methacrylate

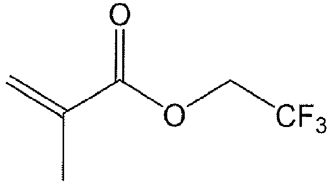

### Compound B1-2: 2,2,2-trifluoroethyl acrylate

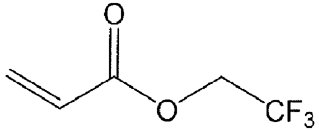

### Compound B1-3: 1,1,1,3,3,3-hexafluoroisopropyl acrylate

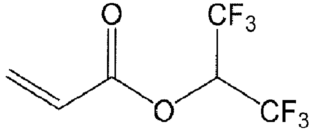

### Compound B2: triallyl isocyanurate

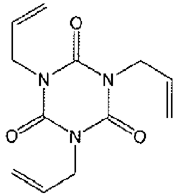

### Compound B3-1: 1,3-bis(isocyanatomethyl)cyclohexane

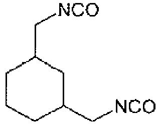

### Compound B3-2: hexamethylene diisocyanate

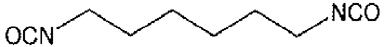

### [Production of Electrodes]

Using a mixer, 50% by mass of SiO as a negative electrode active material, 25% by mass of polyacrylic acid as a binder, and 25% by mass of a carbon black as a conductive material were kneaded to prepare a slurry. The thus obtained slurry was applied and dried onto a 20 µm-thick copper foil by a blade method, and the resultant was roll-pressed using a press machine. The thus obtained negative electrode sheet was punched out in a disk shape of 12.5 mm in diameter to produce a negative electrode. In addition, a lithium metal foil was punched out in a disk shape of 14 mm in diameter to produce a counter electrode.

### [Production of Lithium Secondary Batteries]

Coin-type lithium ion secondary batteries were each produced by laminating the negative electrode produced above, a separator impregnated with each electrolyte solution prepared above, and the counter electrode produced above, and sealing the resultant in a coin-shaped metal container.

### [Charge-Discharge Measurement]

Each battery was stabilized by performing an operation of charging the battery to 5 mV at a current value of 0.1 C in a voltage range of 1.5 V to 5 mV at 25°C, further charging the battery to a current density of 0.01 C with a constant voltage of 5 mV, and then discharging the battery to 1,500 mV at a current value of 0.1 C (this operation is hereinafter referred to as "low-rate test") three times. The discharge capacity in the third operation was defined as "reference capacity". Subsequently, 19 cycles, each of which consisted of charging the battery to 5 mV at 1 C, further charging the battery to a current density of 0.01 C with a constant voltage of 5 mV, and then discharging the battery to 1,500 mV at 1 C followed by evaluation, were performed as a high-rate test. Thereafter, the low-rate test was performed once, and 19 cycles of the high-rate test were further performed. Finally, the low-rate test was performed again, and the discharge capacity in this low-rate test was defined as "discharge capacity after cycle test".

### [Evaluation of Durability]

The cycle capacity retention rate (%) was calculated using the following equation: [(Discharge capacity after cycle test)/(Reference capacity)] × 100. In Examples B2 to B9 and Comparative Examples B1 to B6, the calculated retention rate was normalized such that the retention rate of Example B1 was 100. The results thereof are shown in Table 5.

### [Evaluation of Charging Characteristics]

In the above-described charge-discharge measurement, the time required for charging 50% of the reference capacity in the 19th cycle of the high-rate test was defined as "high-rate charging time". It can be said that the shorter this time, the superior the high-current charging characteristics. In Examples B2 to B9 and Comparative Examples B1 to B6, the measured high-rate charging time was normalized such that the high-rate charging time of Example B1 was 100. The results thereof are shown in Table 5.

**Table 5**

| | Compound (2) | | Specific nitride-containing compound B | | | | Effect | |
|---|---|---|---|---|---|---|---|---|
| | | | Compound (3) | | Compound (4) | | | |
| | Compound | Content (% by mass) | Compound | Content (% by mass) | Compound | Content (% by mass) | Retention rate | High-rate charging time |
| Example B1 | | 0.25 | - | - | Compound B3-1 | 0.25 | 100 | 100 |
| Example B2 | Compound B1-1 | 0.25 | - | - | Compound B3-2 | 0.25 | 98 | 103 |
| Example B3 | Compound B1-1 | 0.25 | Compound B2 | 0.25 | - | - | 85 | 118 |
| Example B4 | Compound B1-2 | 0.25 | - | - | Compound B3-1 | 0.25 | 99 | 103 |
| Example B5 | Compound B1-2 | 0.25 | - | - | Compound B3-2 | 0.25 | 96 | 132 |
| Example B6 | Compound B1-2 | 0.25 | Compound B2 | 0.25 | - | - | 93 | 100 |
| Example B7 | Compound B1-3 | 0.25 | - | - | Compound B3-1 | 0.25 | 100 | 97 |
| Example B8 | Compound B1-3 | 0.25 | - | - | Compound B3-2 | 0.25 | 93 | 100 |
| Example B9 | Compound B1-3 | 0.25 | Compound B2 | 0.25 | - | - | 85 | 153 |
| Comparative Example B1 | Compound B1-1 | 0.25 | - | - | - | - | 75 | 200 |
| Comparative Example B2 | Compound B1-2 | 0.25 | - | - | - | - | 60 | 332 |
| Comparative Example B3 | Compound B1-3 | 0.25 | - | - | - | - | 77 | 197 |
| Comparative Example B4 | - | - | - | - | Compound B3-1 | 0.25 | 75 | 329 |
| Comparative Example B5 | - | - | - | - | Compound B3-2 | 0.25 | 79 | 232 |
| Comparative Example B6 | - | - | Compound B2 | 0.25 | - | - | 72 | 203 |

As apparent from Table 5 above, it is seen that the batteries produced in Examples B1 to B9 had a higher retention rate and a shorter high-rate charging time than the batteries of Comparative Examples B1 to B6. These results indicate that both excellent durability and excellent charging characteristics are attained in a non-aqueous electrolyte secondary battery by using a non-aqueous electrolyte solution having a composition that includes a specific compound (compound (2)), which has an acryloyl group or a methacryloyl group as a partial structure and a fluorine atom-containing hydrocarbon group, and a specific nitrogen-containing compound. From the results, it is seen that the compound (2) and specific nitrogen-containing compound synergistically exert a favorable effect for the durability and the charging characteristics only when they both are incorporated. In the above-described Examples and Comparative Examples shown in Table 5, the cycle test was conducted in a relatively short period as a model; however, significant differences were confirmed. The actual use of a non-aqueous electrolyte secondary battery may extend to several years; therefore, it is understandable that the above-described differences in the results would be more prominent, assuming the use over an extended period.

This application is based on Japanese patent applications filed on October 7, 2019 (Japanese Patent Application Nos. 2019-184550 and 2019-184551), the entirety of which is hereby incorporated by reference.

### INDUSTRIAL APPLICABILITY

The non-aqueous electrolyte solution of the present invention can provide a non-aqueous electrolyte secondary battery with both excellent durability and excellent charging characteristics. Therefore, the non-aqueous electrolyte solution of the present invention and a non-aqueous electrolyte secondary battery obtained using the same can be used in a variety of known applications. Specific examples of the applications of the non-aqueous electrolyte solution of the present invention include laptop computers, stylus computers, portable computers, electronic book players, mobile phones, portable fax machines, portable copiers, portable printers, headphone stereos, video cameras, liquid crystal TVs, handy cleaners, portable CD players, mini-disc players, transceivers, electronic organizers, calculators, memory cards, portable tape recorders, radios, back-up power supplies, motors, automobiles, motorcycles, motor-assisted bikes, bicycles, lighting equipment, toys, gaming machines, watches, power tools, strobe lights, cameras, household backup power sources, backup power sources for commercial use, load leveling power sources, power sources for storing natural energy, and lithium ion capacitors.

## Claims

1. A non-aqueous electrolyte solution, comprising:
a non-aqueous solvent;
a compound represented by the following Formula (1); and
at least one heteroatom-containing lithium salt selected from the group consisting of (A) a lithium salt having an F-S bond, (B) a lithium salt having an oxalic acid skeleton, and (C) a lithium salt having P=O and P-F bonds,
wherein the content of the heteroatom-containing lithium salt is 0.001% by mass or more and 5% by mass or less: 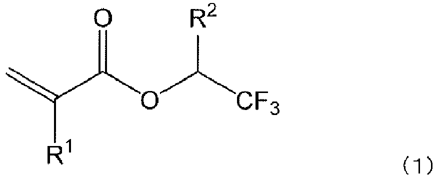 (wherein, R¹ represents a hydrogen atom or a methyl group, and R² represents a hydrogen atom, a halogen atom, or a hydrocarbon group having 1 to 5 carbon atoms and optionally containing a halogen atom).

2. The non-aqueous electrolyte solution according to claim 1, wherein the content of the compound represented by Formula (1) is 0.001% by mass or more and 20% by mass or less.

3. The non-aqueous electrolyte solution according to claim 1 or 2, wherein the content of the heteroatom-containing lithium salt is 0.001% by mass or more and 3% by mass or less.

4. The non-aqueous electrolyte solution according to any one of claims 1 to 3, further comprising at least one selected from the group consisting of LiPF₆, LiBF₄, LiClO₄, and Li(CF₃SO₂)₂N.

5. The non-aqueous electrolyte solution according to any one of claims 1 to 4, further comprising a cyclic carbonate compound having an unsaturated carbon-carbon bond, or a cyclic carbonate compound having a fluorine atom.

6. A non-aqueous electrolyte secondary battery, comprising:
a negative electrode and a positive electrode that are capable of occluding and releasing metal ions; and
a non-aqueous electrolyte solution,
wherein the non-aqueous electrolyte solution is the non-aqueous electrolyte solution according to any one of claims 1 to 5.

7. A non-aqueous electrolyte solution, comprising:
a non-aqueous solvent;
a lithium salt as an electrolyte; and
a compound represented by Formula (2), and at least one of a compound represented by Formula (3) and an isocyanate compound: 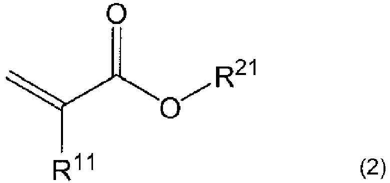 (wherein, R¹¹ represents a hydrogen atom or a methyl group, and R²¹ represents a fluorine atom-containing hydrocarbon group having 1 to 10 carbon atoms) 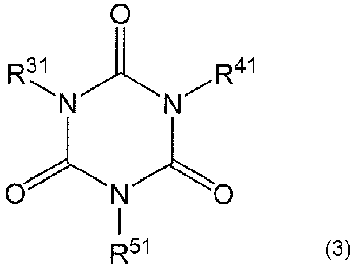 (wherein, R³¹ to R⁵¹ may be the same or different from each other, and each represent an optionally substituted organic group having 1 to 20 carbon atoms).

8. The non-aqueous electrolyte solution according to claim 7, wherein the content of the compound represented by Formula (2) is 0.001% by mass or more and 20% by mass or less.

9. The non-aqueous electrolyte solution according to claim 7 or 8, wherein the content of the compound represented by Formula (3) and/or the isocyanate compound is 0.001% by mass or more and 20% by mass or less.

10. A non-aqueous electrolyte secondary battery, comprising:
a negative electrode and a positive electrode that are capable of occluding and releasing metal ions; and
a non-aqeuous electrolyte solution,
wherein the non-aqueous electrolyte solution is the non-aqueous electrolyte solution according to any one of claims 7 to 9.
